(19) **Europäisches Patentamt**
**European Patent Office**
**Office européen des brevets**

(11) **EP 4 251 221 B1**

(12) **EUROPÄISCHE PATENTSCHRIFT**

(45) Veröffentlichungstag und Bekanntmachung des Hinweises auf die Patenterteilung:
**08.04.2026 Patentblatt 2026/15**

(21) Anmeldenummer: **21819332.4**

(22) Anmeldetag: **11.11.2021**

(51) Internationale Patentklassifikation (IPC):
**A61L 27/52** *(2006.01)*    **H01B 1/12** *(2006.01)*

(52) Gemeinsame Patentklassifikation (CPC):
**A61N 1/0496; A61K 9/0009; A61K 9/06; A61K 47/32; H01B 1/122**

(86) Internationale Anmeldenummer:
**PCT/DE2021/100902**

(87) Internationale Veröffentlichungsnummer:
**WO 2022/111755 (02.06.2022 Gazette 2022/22)**

(54) **VERFAHREN ZUR ERFASSUNG UND BEEINFLUSSUNG EINER AUFNAHME UND/ODER FREIGABE VON BIOAKTIVEN SUBSTANZEN MIT HILFE VON ELEKTRISCH LEITFÄHIGEN HYDROGELEN**

METHOD FOR SENSING AND INFLUENCING AN ABSORPTION AND/OR RELEASE OF BIOACTIVE SUBSTANCES BY MEANS OF ELECTRICALLY CONDUCTIVE HYDROGELS

PROCÉDÉ POUR DÉTECTER ET INFLUER SUR UNE ABSORPTION ET/OU LIBÉRATION DE SUBSTANCES BIOACTIVES AU MOYEN D'HYDROGELS ÉLECTROCONDUCTEURS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.11.2020 DE 102020131547**

(43) Veröffentlichungstag der Anmeldung:
**04.10.2023 Patentblatt 2023/40**

(60) Teilanmeldung:
**25157638.5 / 4 531 059**

(73) Patentinhaber: **Leibniz-Institut für Polymerforschung Dresden e.V.**
**01069 Dresden (DE)**

(72) Erfinder:
• **AKBAR, Teuku Fawzul**
**01099 Dresden (DE)**
• **TONDERA, Christoph**
**04103 Leipzig (DE)**
• **MINEV, Ivan**
**7000 Ruse (BG)**
• **FREUDENBERG, Uwe**
**01217 Dresden (DE)**

• **WERNER, Carsten**
**01324 Dresden (DE)**

(74) Vertreter: **Sperling, Thomas**
**Sperling, Fischer & Heyner Patentanwälte**
**Tolkewitzer Straße 22**
**01277 Dresden (DE)**

(56) Entgegenhaltungen:
**KR-A- 20190 140 217    US-A1- 2020 191 757**
**US-B2- 9 299 476**

• **PIELESZ ANNA ET AL: "[Therapeutically active dressings–biomaterials in a study of collagen glycation]", POLIMERY W MEDYCYNIE - POLYMERS IN MEDICINE - POLYMERS IN DERMEDIZIN, PANSTWOWE WYDAWNICTWO NAUKOWE, WARSAW, PL, vol. 42, no. 2, 1 January 2012 (2012-01-01), pages 115 - 120, XP009532810, ISSN: 0370-0747**
• **PIELESZ ANNA ET AL: "Therapeutically active dressings - biomaterials in a study of collagen glycation", POLIMERY W MEDYCYNIE 2012, vol. 42, no. 2, 2012, pages 115 - 120, XP009532810, ISSN: 0370-0747**

- **KÖNIG R: "ZUR BESCHRÄNKUNG DES ANSPRUCHSINHALTS DURCH "BAR"-DERIVATE", MITTEILUNGEN DER DEUTSCHEN PATENTANWÄLTE, 1997, pages 62, XP001248632, ISSN: 0026-6884**

**Beschreibung**

Die Erfindung betrifft ein Verfahren zur Beeinflussung und Erfassung einer Aufnahme von bioaktiven Substanzen in einem Hydrogelmaterial und/oder einer Freisetzung von bioaktiven Substanzen aus dem Hydrogelmaterial.

Substanzfreisetzungssysteme und Substanzaufnahmesysteme auf Hydrogelbasis sind für Anwendungen in der Biotechnologie und insbesondere in der Medizin vielversprechend, da Hydrogele im Vergleich zu vielen anderen Biomaterialien dem menschlichen physiologischen Gewebe in ihrem Wassergehalt und bezüglich mechanischer Eigenschaften ähnlich sind und einen Einschluss von verschiedenen Substanzen und deren gezielte Freigabe ermöglichen. Bei Hydrogelen handelt es sich definitionsgemäß um stark hydratisierte, kovalent oder physikalisch vernetzte Polymere, welche es erlauben Substanzen über verschiedene, nicht-kovalente Wechselwirkungen reversibel an affine Polymerbausteine des Hydrogelnetzwerks zu binden und somit entweder die Substanzen gezielt aus einem Biofluid oder einem Lebendgewebe zu entfernen, das heißt im Hydrogel zu sequestrieren, oder aus dem Hydrogel an das Biofluid oder das Lebendgewebe freizusetzen. Solche Substanzen können proteinbasierte Signalmoleküle aus der Klasse der Zytokine, Chemokine, Hormone, Neurotransmitter, Wachstumsfaktoren oder nicht-proteinbasierte niedermolekulare Wirkstoffe, sogenannte Small Molecules (engl. kleine Moleküle), welche eine oder mehrere biologische Funktionen der zuvor genannten proteinbasierten Signalmoleküle übernehmen oder auch nicht-proteinbasierte chemische/medizinische Wirkstoffe wie antimikrobielle Wirkstoffe, Antiseptika, Farbstoffe sein, die entweder über Ladungswechselwirkungen und/oder Hydrophobwechselwirkungen oder andere spezifisch, chemische Wechselwirkungen wie Wasserstoffbrückenbindungen, reversibel an die die Affinitätvermittelnde Polymerbausteine im Hydrogel anbinden. Aus dem Stand der Technik sind Hydrogelsysteme bekannt, bei denen die physikochemischen Eigenschaften eines Hydrogels zur Sequestrierung und/oder Freisetzung für bestimmte Substanzen bereits bei der Bildung festgelegt werden müssen. Dies wird durch eine spezifische Ladungscharakteristik des Hydrogelnetzwerks wie beispielsweise aus WO 2018/162009 A2 bekannt, oder durch die gezielte Einstellung von physikalischen Eigenschaften wie Maschenweite erreicht. Es ist außerdem bekannt, kovalent gekoppelte Substanzen durch enzymatische Trigger, Licht oder variable pH-Werte kontrolliert freizusetzen, wobei zum Nachteil derartiger Hydrogelnetzwerke keine reversible Substanzbindung möglich ist. Eine kontrollierte Sequestrierung von Substanzen und somit Abreicherung aus einem Biofluid oder einer Gewebeumgebung ist somit nicht möglich.

Bei den bekannten Systemen, welche die Affinität zu den Substanzen reversibel über nicht-kovalente Wechselwirkungen realisieren, wird die Affinität und somit die Kontrolle der Sequestrierung oder Freisetzung von Substanzen durch ihre intrinsischen, das heißt bei der Bildung des Hydrogelnetzwerks aufgeprägte Netzwerkarchitektur bestimmt. Eine nachträgliche Modulation ist nur über die zuvor genannten externen Trigger Licht, pH-Wert-Änderungen oder enzymatische Spaltung möglich.

Neben intrinsisch geladenen Hydrogelen sind elektrisch leitfähige Hydrogele in der biomedizinischen Forschung beschrieben. Sie werden vorwiegend als Beschichtung für Elektrodenmaterialien, zum Beispiel für neuronale Elektroden verwendet, um deren Bioverträglichkeit und Ladungsinjektion zu optimieren. Aufgrund ihrer mechanischen Ähnlichkeit mit Geweben und Organen führt eine Verwendung von weichen hydratisierten Polymermaterialien im Vergleich zu einer Verwendung von Metallelektroden zu geringeren Abstoßungsreaktionen. Zusätzlich findet bei klassischen Metallelektroden die Ladungsinjektion, welche für die Stimulation von Geweben und Organen essentiell ist, nur an der Oberfläche der Elektroden statt. Durch Nanostrukturierung wird versucht, die Oberfläche und damit die Kontaktfläche zwischen der Elektrode und der Gewebeflüssigkeit (Biofluid) zu erhöhen, was eine erhöhte Ladungsinjektion zur Folge hat. Da das Biofluid bei elektrisch leitfähigen Hydrogelen in das gesamte Volumen des Materials eindringen kann, ist die Kontaktfläche bereits ohne weitere Strukturierung deutlich größer als bei Metallelektroden mit vergleichbarer Größe. Dies führt ebenfalls zu einer deutlich gesteigerten Ladungsinjektion. Als Maß für die Fähigkeit Ladungen in physiologische Lösungen zu übermitteln wird in der Literatur der Begriff Ladungsspeicherkapazität (englisch *charge storage capacity*) verwendet.

Zur Herstellung solcher leitfähigen Hydrogele werden leitfähige Substanzen wie Kohlenstoffnanoröhren und/oder elektrisch leitfähige Polymere verwendet, wobei zumeist die leitfähige Substanz in einer Hydrogel-Präkursorlösung verteilt und anschließend das Hydrogel durch Polymerisation und Vernetzung in einer weiteren von der leitfähigen Substanz unabhängigen Komponente gebildet wird. Hierbei ist als übergreifende Eigenschaft zu nennen, dass die elektrisch leitfähige Komponente zumeist hydrophob ist und daher kein Hydrogel bildet.

Aufgrund ihrer vielfältigen Einsatzmöglichkeiten und ihrer einfachen und kostengünstigen Verarbeitung werden leitfähige organische Polymere zunehmend in industriellen Anwendungen genutzt. Die Polymere arbeiten nach dem Prinzip von Halbleitern. Wichtigster Bestandteil ist ein konjugiertes $\pi$-Elektronensystem, welches sich durch das gesamte Polymerrückgrat erstreckt. Die intrinsische elektrische Leitfähigkeit dieser Polymere ist gering. Um freie Ladungsträger zu erzeugen, mit dem Ziel, die molekulare elektrische Leitfähigkeit zu steigern, werden geladene Moleküle benötigt, die Elektronen aus dem Polymersystem entfernen (p-Dotierung) oder Elektronen in das Polymersystem einbringen (n-Dotierung). Dieser Prozess wird als primäre Dotierung bezeichnet.

Die meist verwendete Methode ist die p-Dotierung. Die Dotierung und daraus resultierende Interaktion von hydrophoben Polymeren mit stark geladenen hydrophilen Molekülen reduziert zusätzlich die Hydrophobizität der Polymer-Dotierungs-Komplexe und erlaubt damit die Verwendung dieser Polymere in wässrigen Lösungen oder Hydrogelen.

Neben der primären Dotierung ist auch die Anordnung der leitfähigen Polymerketten zueinander von entscheidender Bedeutung. Für den Transport von Ladungsträgern über längere Strecken müssen diese zwischen den elektrisch leitfähigen Polymerketten übertragen werden. Dafür ist eine gleichmäßige Verteilung der Polymerketten im Volumen bei gleichzeitiger räumlicher Nähe zueinander notwendig. Wässrige Lösungen von leitfähigen Polymeren und dem jeweiligen dotierenden Molekül bilden meist Suspensionen. Durch die fehlende Interaktion der leitfähigen Polymerketten untereinander resultiert eine geringe elektrische Leitfähigkeit. Durch die strukturelle Neuorganisation der leitfähigen Polymerketten zueinander (sekundäre Dotierung) kann die notwendige räumliche Nähe der Polymerketten zueinander erreicht und dadurch die Leitfähigkeit weiter gesteigert werden. Dies kann zum Beispiel durch das Erhöhen der Ionenstärke und der damit verbunden Abschirmeffekte der Ladung (charakterisiert über die Debye-Länge) erreicht werden.

Ein Beispiel mit dem leitfähigem Poly-3,4-ethylendioxythiophen (PEDOT) wird von Zhenan Bao und weiteren Autoren beschrieben (DOI: 10.1038/s41467-018-05222-4, und US20190390068A1). Wie aus dieser Schrift bekannt ist, wird aus einer Mischung von hydrophilen Polystyrensulfonat (PSS) und PEDOT (primäre Dotierung) durch physikalische Verschlaufungen des PSS und unter Verwendung von ionischen Flüssigkeiten (sekundäre Dotierung) zunächst ein schwach vernetztes Hydrogel gebildet, welches im Anschluss durch Bildung eines mit radikalischer Vernetzungsreaktion von Acrylatmonomeren gebildetes pseudo-interpenetrierendes Netzwerk mechanisch stabilisiert wird. Nach der Polymerisation resultiert ein elektrisch leitfähiges Hydrogel-Netzwerk (10.1038/s41467-018-05222-4, und US20190390068A1), wobei das sekundäre Polymer-Netzwerk lediglich der Stabilisierung und Verbesserung der mechanischen Eigenschaften des Materials dient. Die elektrischen Eigenschaften des Hydrogels resultieren allein aus dem primären, nicht kovalenten PEDOT:PSS Hydrogel.

Eine weitere Methode elektrisch leitfähige Hydrogele zu erhalten ist aus US 9 299 476 B2 bekannt. Dem aus US 9 299 476 B2 bekannten Verfahren zufolge wird ein auf Biopolymeren basierendes Hydrogelnetzwerk, welches funktionelle anionische Gruppen trägt, auf der Oberfläche einer Elektrode gebildet und abgeschieden. Das so erhaltene primäre Netzwerk wird in einer 3,4-Ethylendioxythiophen (EDOT)-Monomere enthaltenden Lösung gequollen beziehungsweise in diese überführt und eine elektrische Polymerisation der EDOT-Monomere zu Poly-3,4-ethylendioxythiophen (PEDOT) durch den Einfluss einer elektrischen Spannung ausgelöst. Es entsteht ein pseudo-interpenetrierendes Polymernetzwerk durch die ionische Interaktion des PEDOT mit den anionischen funktionellen Gruppen des primären Netzwerkes. Diese anionischen Gruppen wirken gleichzeitig p-dotierend auf das PEDOT (primäre Dotierung) und erzeugen in Kombination ein elektrisch leitfähiges Hydrogelmaterial. Aufgrund der vorgegebenen Verteilung der kovalent im Hydrogel gebundenen anionischen Gruppen und der nachträglichen Polymerisation des PEDOT um das primäre Hydrogelnetzwerk, können aufgrund der gleichmäßigen Verteilung der PEDOT Polymerketten, bereits ohne sekundäre Dotierung Hydrogele mit hoher elektrischer Leitfähigkeit erhalten werden.

Die bisher bekannten Hydrogelmaterialien ermöglichen eine Sequestrierung und/oder Freisetzung von Substanzen durch die intrinsisch aufgeprägte physikochemische Charakteristik oder zeigen im Falle von elektrisch leitfähigen Hydrogelmaterialien eine eingeschränkte Modulierbarkeit und Spezifizität für die Wechselwirkung mit bestimmten bioaktiven Substanzen.

US2020/0191757 offenbart die Bestimmung der Konzentration von Metallionen in einem Biofluid durch Messung einer elektrischen Widerstandsänderung eines mit dem Biofluid in Kontakt gebrachten Hydrogels.

Es ist Aufgabe der Erfindung, ein Verfahren vorzuschlagen, mit welchem Konzentrationen von bioaktiven Substanzen in einem Hydrogelmaterial oder in einer Umgebung eines Hydrogelmaterials beeinflusst und bestimmt werden können.

Die Aufgabe wird durch ein Verfahren mit den Merkmalen gemäß Patentanspruch 1 gelöst. Weiterbildungen sind in den abhängigen Patentansprüchen angegeben.

Die Erfindung umfasst ein Verfahren zur Erfassung und Beeinflussung einer Aufnahme von bioaktiven Molekülen in einem Hydrogelmaterial und/oder Freisetzung von bioaktiven Molekülen aus dem Hydrogelmaterial, wobei das Hydrogelmaterial definitionsgemäß ein aus anionisch geladenen Bausteinen und ungeladenen Bausteinen ausgebildetes Polymernetzwerk ist, dessen Affinität für bioaktive Moleküle anhand von die anionisch geladenen Bausteine definierenden Parametern konfigurierbar ist und eine elektrisch leitfähige Komponente aufweist, deren elektrischer Widerstand und elektrische Ladungsspeicherkapazität von einer Interaktion mit Hydogel-Bausteinen und einer Bindung bioaktiver Moleküle an das Hydrogelmaterial abhängt, wobei die elektrisch leitfähige Komponente geeignet ist, die anionische Ladung des Hydrogelmaterials und dessen Affinität für bioaktive Moleküle durch den Einfluss eines elektrischen Potentials, zu verändern. Bei dem Verfahren wird das vordefinierte Hydrogelmaterial mit einem Biofluid in Kontakt gebracht, wobei eine Änderung des elektrischen Widerstandes und/oder eine Änderung der Ladungsspeicherkapazität des Hydrogelmaterials erfasst und anhand der erfassten Änderung des elektrischen Widerstandes und und/oder der erfassten Ladungsspeicherkapazitätsänderung eine Aufnahme von bioaktiven Molekülen in das Hydrogelmaterial oder eine Freisetzung von bioaktiven Molekülen aus dem Hydrogelmaterial in das Biofluid ermittelt wird, und/oder wobei eine Konzentration von bioaktiven Molekülen in dem Biofluid und/oder eine Konzentration von bioaktiven Molekülen in dem Hydrogelmaterial durch ein auf das Hydrogelmaterial wirkendes elektrisches Potential beeinflusst wird.

Das aus anionisch geladenen Bausteinen und ungeladenen Bausteinen gebildete Polymernetzwerk ist ein anionisch geladenes Polymernetzwerk. Das anionisch geladene Polymernetzwerk ist anhand von Parametern, welche die anionisch geladenen Bausteine definieren, konfigurierbar.

Das definitionsgemäße Hydrogelmaterial, welches elektrisch leitfähig ist, wird der Einfachheit halber nachfolgend als Hydrogelmaterial bezeichnet.

Als Biofluid sind im Sinne der Erfindung physiologische Lösungen, Zellkulturen und Lebendgewebe zu verstehen. Die Handlungsanweisung Kontaktieren des Hydrogelmaterials mit einem Biofluid kann daher als eine Kontaktierung durch Eintauchen in eine physiologische Lösung oder als eine Flächenkontaktierung des Hydrogelmaterials mit einem Lebendgewebe *in vivo* und *in vitro* verstanden werden.

Als bioaktive Moleküle werden im Sinne der Erfindung proteinbasierte und nicht proteinbasierte bioaktive Substanzen, Wirkstoffe sowie Small Molecules, welche Signaleigenschaften von Zytokinen, Chemokinen, Hormonen, Neurotransmittern oder Wachstumsfaktoren aufweisen sowie weitere biologische Wirkungen verursachen, verstanden. Bioaktive Moleküle können insbesondere pharmazeutische Wirkstoffe sein. Für alle zuvor genannten bioaktiven Moleküle gilt als übergreifendes Merkmal ein Molekulargewicht von kleiner gleich 70 kDa.

Das erfindungsgemäße Verfahren umfasst eine Erfassung einer Aufnahme von bioaktiven Substanzen in einem Hydrogelmaterial und/oder Freisetzung von bioaktiven Molekülen aus dem Hydrogelmaterial sowie eine Beeinflussung einer Aufnahme von bioaktiven Molekülen in einem Hydrogelmaterial und/oder Freisetzung von bioaktiven Molekülen aus dem Hydrogelmaterial. Es kann somit eine Erfassung und Beeinflussung von bioaktiven Molekülen oder - gemäß der Alternative - eine Erfassung oder Beeinflussung von bioaktiven Molekülen durchgeführt werden.

Dass zur Erfassung einer Aufnahme von bioaktiven Molekülen in dem Hydrogelmaterial und/oder einer Freisetzung von bioaktiven Molekülen aus dem Hydrogelmaterial eine Änderung des elektrischen Widerstands des Hydrogelmaterials und/oder eine elektrische Ladungskapazitätsänderung des Hydrogelmaterials erfasst wird/werden, liegt der Erkenntnis zugrunde, dass die Änderung des elektrischen Widerstands des Hydrogelmaterials und/oder eine elektrische Ladungskapazitätsänderung des Hydrogelmaterials durch die Bindung bioaktiver Moleküle an das Hydrogelmaterial beeinflusst wird. So wird der elektrische Widerstand des Hydrogelmaterials infolge der Sequestrierung und Bindung an oder in dem Hydrogelmaterial erhöht, wobei die elektrische Leitfähigkeit des Hydrogelmaterials durch die Beladung der anionischen Gruppen mit bioaktiven Molekülen sinkt. Im Umkehrschluss wird der elektrische Widerstand des Hydrogelmaterials verringert, wenn bioaktive Moleküle aus oder von dem Hydrogelmaterial gelöst werden, wobei die elektrische Leitfähigkeit des Hydrogelmaterials ansteigt.

Die Berechnung der Ladungsspeicherkapazität erfolgt anhand der Messung der zyklischen Voltammetrie. Dafür wird in einem 3 Elektroden-Aufbau der Stromfluss zwischen Arbeitselektrode (Hydrogelmaterial) und Gegenelektrode (Kohlenstoff-Elektrode) gemessen, während in 5 zyklischen Durchläufen das angelegte elektrische Potential von -0,6 bis 0,8 V (Potential zwischen Arbeitselektrode und Ag/AgCl-Referenzelektrode) variiert wird. Die Scan-Rate beträgt 50 mV/s. Anschließend wird der negative Teil der Fläche unter der Kurve integriert (MultiTrace 4.3, PalmSens 4) und daraus die Ladungsspeicherkapazität mit nachfolgender Formel berechnet:

$$Ladungsspeicherkapazit\ddot{a}t = \frac{Stromst\ddot{a}rke\ I * Spannung\ U}{Scan - Rate}$$

Als Integral gibt die Software den Wert I * U in der Einheit [A] * [V] aus. Dabei gilt [A] = [C/s]. Durch die Division der Scan-Rate [V/s] resultiert die Ladung [C], welche durch das Material übertragen wird. Diese wird anschließend in das Verhältnis zum Volumen gesetzt, da die Grenzfläche zum umgebenden Medium das komplette Volumen des Hydrogels betrifft und dadurch eine Berechnung der Oberfläche/Kontaktfläche nicht möglich ist.

Zur Erfassung einer Aufnahme von bioaktiven Molekülen in dem Hydrogelmaterial und/oder einer Freisetzung von bioaktiven Molekülen aus dem Hydrogelmaterial kann alternativ eine Änderung der elektrischen Leitfähigkeit des Hydrogelmaterials bestimmt werden, wobei anhand der Änderung der elektrischen Leitfähigkeit eine Aufnahme von bioaktiven Molekülen in dem Hydrogelmaterial und/oder eine Freisetzung von bioaktiven Molekülen aus dem Hydrogelmaterial ermittelt wird. Die Bindung bioaktiver Moleküle im elektrisch leitfähigen Hydrogelmaterial erfolgt über nichtkovalente Wechselwirkungen der bioaktiven Moleküle mit den Polymerketten des Hydrogelmaterials. Dabei spielen insbesondere ionische Wechselwirkungen eine wichtige Rolle. Durch die Interaktion von anionischen und kationischen Gruppen der gebundenen bioaktiven Moleküle mit dem anionische Gruppen tragenden Polymernetzwerk des Hydrogelmaterials sowie dem kationischen, elektrisch leitfähigen Polymer, erfolgt eine Änderung der Dotierung des leitfähigen Polymers. Die anionischen Gruppen in den gebundenen Molekülen können dabei zur Dotierung beitragen und die Leitfähigkeit erhöhen. Kationische Gruppen hingegen kompensieren negative Ladungen der anionischen Polymerkomponente und haben dadurch einen negativen Einfluss auf die Dotierung. Zusätzlich können hydrophobe Bereiche der biokativen Substanzen die Interaktion der PEDOT Polymerketten zueinander beeinflussen. In Abhängigkeit von dem Molekültyp der gebundenen bioaktiven Moleküle sowie der Konzentration der bioaktiven Moleküle findet somit eine spezifische Änderung der elektrischen Eigenschaften des Hydrogelmaterials statt.

Eine Aufnahme und/oder Freisetzung von bioaktiven Molekülen kann als Konzentrationsänderung von bioaktiven Molekülen in dem Hydrogelmaterial verstanden werden. Ferner kann vorgesehen sein, dass einem diskreten Leitfähigkeitswert, einem diskreten elektrischen Widerstandswert oder einem diskreten Wert der elektrischen Ladungsspeicherkapazität eine diskrete Konzentration eines bioaktiven Moleküls zugeordnet wird.

Der elektrische Widerstand des Hydrogelmaterials kann als Gleichstromwiderstand oder als Impedanz ermittelt werden. Für die Impedanzerfassung kann ein Frequenzbereich von 0,01 Hz bis 1 MHz vorgegeben sein. Es kann vorgesehen werden, dass zur Erfassung einer Bindung von bioaktiven Molekülen an oder in dem Hydrogel eine Veränderung der Impedanz des Hydrogelmaterials bei zumindest einer Frequenz im Bereich von 0,1 Hz bis 1 MHz gemessen wird. Dabei kann weiterhin eine Veränderung der Ladungsspeicherkapazität berücksichtigt werden.

Zur Beeinflussung einer Konzentration von bioaktiven Molekülen in dem Biofluid und/oder einer Konzentration von bioaktiven Molekülen in dem Hydrogelmaterial wird das Hydrogelmaterial mit einem elektrischen Potential beaufschlagt. Durch den Einfluss eines elektrischen Potentials auf das Hydrogelmaterial wird die anionische Ladung des Hydrogelmaterials und dessen Affinität für bioaktive Moleküle geändert, so dass infolge einer Potentialänderung eine Bindung von bioaktiven Molekülen in oder an dem Hydrogelmaterial beeinflusst wird.

Eine Freisetzung von bioaktiven Molekülen aus dem Hydrogelmaterial setzt voraus, dass bioaktive Moleküle vor der Kontaktierung mit dem Biofluid an oder in dem Hydrogelmaterial gebunden ist. Es kann daher vorgesehen werden, dass zur Freisetzung von bioaktiven Molekülen das Hydrogelmaterial vor der Kontaktierung mit dem Biofluid elektrisch oder chemisch mit einer vorgegebenen Konzentration der vorgegebenen bioaktiven Moleküle beladen wird.

Gemäß einer Ausführungsvariante des erfindungsgemäßen Verfahrens ist vorgesehen, dass das Polymernetzwerk in seiner Zusammensetzung anhand von mindestens drei die anionisch geladenen Bausteine definierenden Parametern, ausgewählt aus einer Gruppe von Parametern P0, P1, P2, P3, konfigurierbar ist, wobei der Parameter P0 einem Wert aus der Anzahl der ionisierten, anionischen Gruppen, unter Annahme einer 30%igen Ionisierung aller anionischen Gruppen, je Volumeneinheit des unter physiologischen Bedingungen gequollenen Hydrogelmaterials entspricht, der Parameter P1 einem Wert aus der Anzahl der stark anionischen Gruppen, mit einem intrinsischen pKs-Wert kleiner als 2,5, je Volumeneinheit des unter physiologischen Bedingungen gequollenen Hydrogelmaterials entspricht, der Parameter P2 einem Wert aus der Anzahl der stark anionischen Gruppen, mit einem intrinsischen pKs-Wert kleiner als 2,5, je Wiederholeinheit geteilt durch die Molmasse der Wiederholeinheit entspricht und der Parameter P3 einem Wert zur Beschreibung der Amphiphilie der anionischen Bausteine entspricht, wobei ein elektrischer Widerstand und/oder eine elektrische Ladungsspeicherkapazität des Hydrogelmaterials durch Parameterwerte einer Parameterkonfiguration des Hydrogelmaterials vorgegeben wird. Eine detaillierte Definition der Parameterwerte und deren Bestimmung ist der Beschreibung weiter unten zu entnehmen.

Eine Wechselwirkung von bioaktiven Molekülen kann weiterhin auf dem substanzspezifischen Wert Pp beruhen, welcher aus dem Verhältnis der Nettoladung eines bioaktiven Moleküls und der für Wasser zugänglichen Oberfläche des bioaktiven Moleküls berechnet wird. Für proteinbasierte Substanzen ist eine beliebige Proteinstruktur in der Protein Data Bank (PDB, http://www.rcsb.org/) verfügbar. Die Nettoladung der ausgewählten Proteinstruktur wird mit dem Delphi Web Server (http://compbio.clemson.edu/sapp/delphi_webserver/) mit Standardeinstellungen bei pH 7 berechnet. Die dem Lösungsmittel Wasser zugängliche Proteinoberfläche wird mit der PyMol-Software (www.pymol.org) unter Nutzung von einem Lösungsmittelradius von Wasser von 1.4 Å berechnet. Dann erfolgt die Berechnung von Pp aus der Nettoladung dividiert durch die dem Lösungsmittel Wasser zugängliche Proteinoberfläche multipliziert mit einem Faktor von 1000000, um die Einheit $10^{-6}$ x $[1/A^2$ bzw. $A^{-2}]$ zu erhalten. Für nicht-proteinbasierte Substanzen wird die aus der chemischen Struktur ableitbare Nettoladung, welche dem Überschuss anionischer oder kationischer Gruppen entspricht, und die durch das Lösungsmittel Wasser zugängliche Moleküloberfläche, welche in Analogie zur Bildungsvorschrift für den Parameter P3 durch Nutzung der ChemDraw19.0 und ChemAxon MarvinSketch 19.21 Software abgeleitet wurde, berechnet. Der erhaltene Wert wird mit einem Faktor von 1000000 multipliziert, um die Einheit $10^{-6}$ x $[1/A^2$ bzw. $A^{-2}]$ zu erhalten. Eine Beladung des Hydrogelmaterials, also die Immobilisierung einer

vorgegebenen Konzentration bioaktiver Moleküle in oder an dem Hydrogelmaterial, kann auf unterschiedliche Weise erfolgen. Gemäß einer ersten Methode, die auch als erste Beladungsmethode bezeichnet werden kann, wird eine vorgegebene Konzentration der zur Bindung vorgesehenen bioaktiven Moleküle mit den anionisch geladenen Hydrogelbausteinen gemischt und dabei in das Polymernetzwerk integriert. Bei der nachfolgenden Bildung der elektrisch leitfähigen Hydrogelmaterialien durch Inkorporation der elektrisch leitfähigen Komponente, sind die bioaktiven Moleküle dann bereits gemäß der vorgegebenen Konzentration in dem Polymernetzwerk enthalten. Bei diesem Vorgehen ist von Vorteil, dass die gesamte Menge der bioaktiven Moleküle nach der Polymernetzwerkbildung quantitativ im Hydrogelmaterial enthalten ist. Mit anderen Worten, die Beladung der bioaktiven Moleküle erfolgt unabhängig von den Parametern P0, P1, P2, P3 und unabhängig von dem substanzspezifischen Wert Pp. Die bei der Hydrogelmaterialbildung herrschenden Reaktionsbedingungen können einen ungünstigen Einfluss auf die Struktur der bioaktiven Moleküle haben, weshalb nicht alle bioaktiven Moleküle für eine Immobilisierung nach der ersten Beladungsmethode geeignet sind.

Gemäß einer zweiten Methode, die auch als zweite Beladungsmethode bezeichnet werden kann, wird das elektrisch leitfähige Hydrogelmaterial mit einer vorgegebenen Parameterkonfiguration und vorgegebenen Parameterwerten gebildet und das elektrisch leitfähige Hydrogelmaterial im Anschluss mit einer wässrigen Lösung oder einem Biofluid als Beladungslösung mit einer vorgegebenen Molekülkonzentration für eine vorgegebene Zeitdauer in Kontakt gebracht. Dabei werden die in Lösung befindlichen bioaktiven Moleküle aus der wässrigen Lösung oder dem Biofluid in Abhängigkeit der Parameter P0, P1, P2, P3 in das Hydrogelmaterial aufgenommen und gebunden. Abschließend wird das beladene Hydrogelmaterial aus der Beladungslösung entfernt. Die an oder in dem Hydrogelmaterial immobilisierten bioaktiven Moleküle können dann durch den Einfluss eines elektrischen Potentials an eine Umgebung, vorzugsweise ein Biofluid oder ein Lebendgewebe, freigesetzt werden. Dabei besteht durch den Einfluss des elektrischen Potentials weiter die Möglichkeit, dass bioaktive Moleküle aus der Umgebung des Hydrogelmaterials in das Hydrogelmaterial sequestriert werden.

Gemäß einer dritten Methode zur Beladung, welche auch als dritte Beladungsmethode bezeichnet werden kann, wird ein vorgegebenes elektrisch leitfähiges Hydrogelmaterial, welches eine vorgegebene Parameterkonfiguration mit vorgegebenen Parameterwerten aufweist, mit einer wässrigen Lösung oder einem Biofluid als Beladungslösung mit einer vorgegebenen Molekülkonzentration für eine vorgegebene Zeitdauer in Kontakt gebracht und dabei dem Einfluss eines elektrischen Potentials ausgesetzt. Dabei werden bioaktive Moleküle in Abhängigkeit der vorgegebenen Parameterwerte aus der Beladungslösung in das Hydrogelmaterial aufgenommen (sequestriert). Es kann erforderlich sein, dass das auf das Hydrogelmaterial wirkende Potential aufrecht erhalten werden muss, um die Bindung der bioaktiven Moleküle konstant zu halten. Andernfalls, wenn das Potential beziehungsweise der Strom verändert wird, können die bioaktiven Moleküle aus dem Hydrogelmaterial freigesetzt werden. Wesentlich ist dabei, dass die Sequestrierung oder Freigabe von bioaktiven Molekülen neben der Beeinflussung durch ein elektrisches Potential auch auf einer durch die Paramter P0, P1, P2, P3, Pp vorgegebenen Affinität des Hydrogelmaterials für bestimmte bioaktive Moleküle beruht.

Es hat sich gezeigt, dass eine die elektrische Leitfähigkeit des Hydrogelmaterials beeinflussende Strukturbildung und Verteilung der elektrisch leitfähigen Komponente in dem Hydrogelmaterial durch die Parameterwerte einer Parameterkonfiguration beeinflusst werden kann. Es kann daher vorgesehen werden, dass ein Hydrogelmaterial vorgegeben wird, welches eine vorgegebene elektrische Leitfähigkeit aufgrund einer vorgegeben Parameterkonfiguration mit vorgegeben Parameterwerten aufweist.

Bei dem Verfahren kann weiter vorgesehen sein, dass das Hydrogelmaterial zur Aufnahme von bioaktiven Molekülen mit einem elektrischen Potential, gegenüber einer Ag/AgCl Referenzelektrode, im Bereich von 1 mV bis 1000 mV, vorzugsweise im Bereich von 400 mV bis 600 mV beaufschlagt wird. Zur Freigabe von bioaktiven Molekülen kann das Hydrogelmaterial mit einem elektrischen Potential, gegenüber einer Ag/AgCl Referenzelektrode, im Bereich von -1 mV bis -1000 mV, vorzugsweise in einem Bereich von -400 mV bis -600 mV beaufschlagt werden.

Das Hydrogelmaterial kann zur Aufnahme von bioaktiven Molekülen mit einem konstanten elektrischen Strom größer als 0 mA beaufschlagt werden, wobei die Richtung des elektrischen Stromflusses zur Freigabe von bioaktiven Molekülen geändert wird.

Weitere Einzelheiten, Merkmale und Vorteile von Ausgestaltungen der Erfindung ergeben sich aus der nachfolgenden Beschreibung von Ausführungsbeispielen. mit Bezugnahme auf die zugehörigen Zeichnungen und Tabellen. Es zeigen:

Figur 1: eine schematische Darstellung zur Erläuterung des Hydrogelmaterials,
Figur 2: eine weitere schematische Darstellung zur weiteren Erläuterung der Erfindung,

Anionisch geladene Hydrogelbausteine sind nachfolgend mit GB abgekürzt, wobei unterschiedliche anionisch geladene Bausteine zusätzlich mit einer Ziffer gekennzeichnet sind. Ungeladene Bausteine des Hydrogelmaterials sind mit UGB abgekürzt, wobei unterschiedliche ungeladene Bausteine mit einer Ziffer gekennzeichnet sind. Der Einfachheit halber und aus Platzgründen sind Hydrogelmaterialien in den Tabellen als Hydrogele bezeichnet. Hydrogelmaterialtypen sind in den Tabellen als Hydrogeltypen bezeichnet.

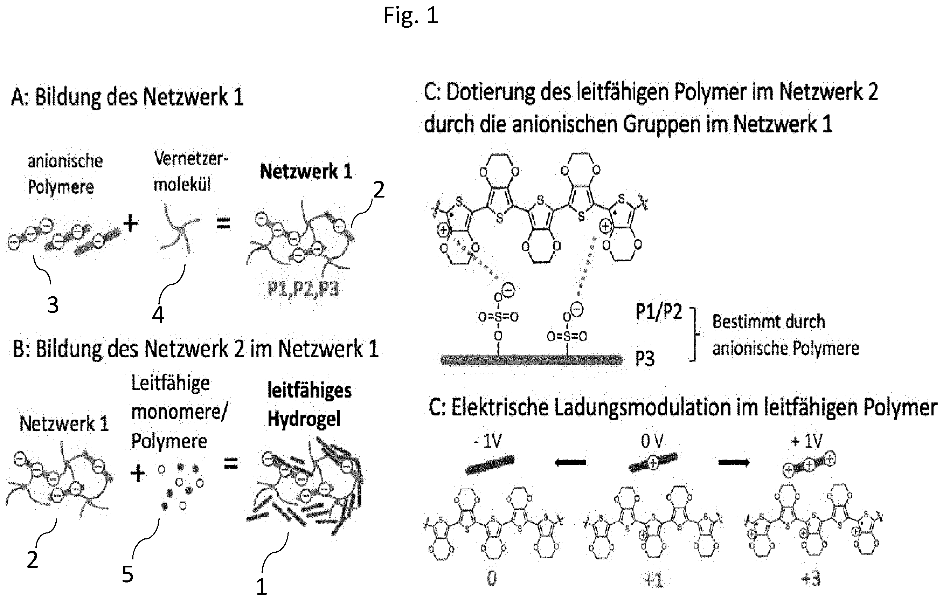

Die **Figur 1** zeigt eine schematische Darstellung zur Erläuterung des elektrisch leitfähigen Hydrogelmaterials 1. Abbildung A der Figur 1 zeigt beispielhaft die Synthese des Polymernetzwerks 2 aus anionisch geladenen Bausteinen 3 und Vernetzermolekülen 4 als Vorlage zur Bildung des Hydrogelmaterials 1. Mit dem Polymernetzwerk 2 wird die Parameterkonfiguration mit den Paremetern P1, P2 und P3 vorgegeben. Die Abbildung B zeigt den Aufbau des elektrisch leitfähigen Hydrogelmaterials 1 als Pseudo-interpenetrierendes Polymernetzwerk (IPN) aus dem Polymernetzwerk 2 und einer elektrisch leitfähigen Komponente 5. Das Polymernetzwerk 2 trägt anionische Gruppen und wird durch die Parameter P1, P2, und P3 bestimmt. Das elektrisch leitfähige Hydrogelmaterial 1 entsteht durch Polymerisation oder Vernetzung des Polymernetzwerks 2 mit der elektrisch leitfähigen Komponente 5. In der Abbildung C der Figur 1 ist die Dotierung der leitfähigen Komponente 5 beispielhaft gezeigt. Im Beispiel ist die elektrisch leitfähige Komponente 5 PEDOT: Die Dotierung erfolgt über die Wechselwirkung mit sulfat-/sulfonat-Gruppen im anionisch geladenen Polymernetzwerk 2. Für die Wechselwirkung mit PEDOT 5 und die dadurch die resultierenden elektrischen Eigenschaften des leitfähigen Hydrogelmaterials 1 sind die integrale Ladungsdichte P1 des Polymernetzwerks 2, die lokale Ladungsdichte P2 und die Hydrophobizität P3 des anionische Gruppen tragenden Polymers im Polymernetzwerk 2 entscheidend. In Abbildung D ist beispielhaft dargestellt, welchen Einfluss das Anlegen eines elektrischen Potentials auf das elektrisch leitfähige Hydrogelmaterial hat. Durch den Einfluss eines elektrischen Potentials kann die positive Ladung des PEDOT stufenlos reguliert werden. Die Regulierung erfolgt dabei von neutral (0) über mittel (+1) bis stark (+3) positiv geladen.

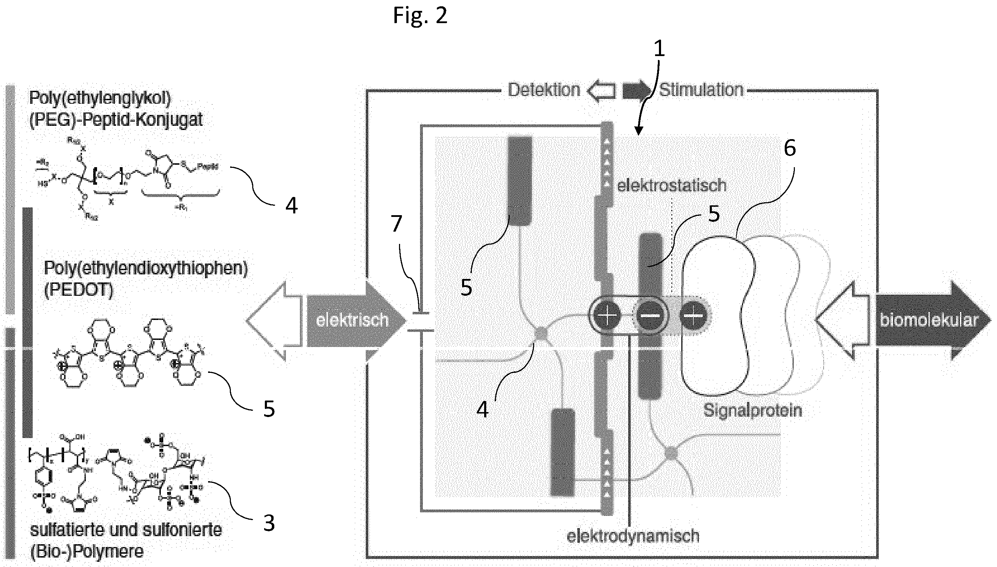

Die **Figur 2** zeigt eine weitere schematische Darstellung zur weiteren Erläuterung der Erfindung. Dargestellt ist ein elektrisch leitfähiges Hydrogelmaterial 1, welches aus sulfatierten und sulfonierten Polymeren als geladende Bausteine 3 und PEG als Vernetzer 4, welche ein Polymernetzwerk 2 bilden, und PEDOT als elektrisch leitfähige Komponente 5, welche in dem Polymernetzwerk inkorporiert ist, ausgebildet ist. Rechts mit dem Bezugszeichen 6 ist ein positiv geladenes Signalprotein dargestellt, welches an negativ geladenem PEDOT gebunden ist. Die Bindungseigenschaften des Signalproteins 6 in dem Hydrogelmaterial 1 ist durch den Einfluss eines elektrischen Potentials beeinflussbar. Zur elektrischen Beeinflussung des Hydrogelmaterials 1 ist das Hydrogelmaterial 1 mit einer Spannungsquelle 7 elektrisch kontaktiert. Das Hydrogelmaterial 1 ermöglicht eine elektrodynamische Modulation spezifischer elektrostatischer Wechselwirkungen zwischen dem Hydrogelpolymeren und dem Signalprotein 6.

Ausführungsbeispiele:

## Synthese des Polymernetzwerks

Für die Synthese des Hydrogelmaterials 1 wurden drei anionisch geladene Polymernetzwerksysteme 2, (1) ein aus zwei-Hydrogelbausteinen, eines anionisch geladenen Bausteins (GB1-5) mit Maleimid- und eines ungeladenen Bausteins (UGB2) mit Thiolgruppen (im weiteren bezeichnet als Maleimid-Thiol Zwei-Komponenten-System) oder (2) ein aus drei Hydrogelbausteinen (ein anionisch geladener Baustein (GB1-5) mit Maleimidgruppen und ein ungeladener Baustein mit Maleimidgruppen (UGB1)) und ein ungeladener Baustein (UGB2) mit Thiolgruppen (im weiteren bezeichnet als Maleimid-Thiol Drei-Komponenten-System) oder (3) ein System aus zwei Hydrogelbausteinen vernetzt über EDC/NHS-basierte Aktivierung der Carboxylgruppen des anionisch geladenen Hydrogelbausteins (GB1) und Reaktion mit den Aminogruppen am zweiten, ungeladenen Hydrogelbaustein (UGB3) (im weiteren als EDC/NHS-System bezeichnet), verwendet.

Die Eigenschaften der anionisch geladenen Hydrogelbausteine (GB1 bis GB5) und der ungeladenen Hydrogelbausteine (UGB1 bis UGB3) sind **Tabelle 1** zu entnehmen.

## Maleimide-Thiol Zwei-Komponenten-System zur Herstellung eines anionisch geladenen Polymernetzwerks als Vorstufe des erfindungsgemäßen Hydrogelmaterials

GB1 (Heparin-Maleimid, 15 kDa) und UGB2 (sternförmiges Thiolfunktionalisiertes Polythylenglycol, starPEG, 10 kDa) werden in 0,1 x phosphatgepufferter Kochsalzlösung ((PBS), pH = 6) in einer Konzentration von je 0,0015 mol/l gelöst. 1 x PBS besteht aus 137 nm NaCl, 2,7 mM KCl und 12 mM Gesamt-Phosphat bestehend aus $HPO_4^{2-}$ und $H_2PO_4$. Die Mischungsverhältnisse und Konzentrationen sind der aus der Tabelle 2 zu entnehmen. Alle nachfolgenden Schritte bis zum Mischen der Hydrogelbausteine erfolgen auf Eis. (pH-Werteinstellung zum Erreichen einer Gelierungszeit von 30min) Für ein molares Verhältnis von 1 beider Bausteine werden gleiche Volumen der beiden Lösungen durch Vermischung mittels einer Pipette und/oder in einem Mischgerät gemischt. Anschließend wird die Mischung zentrifugiert um Luftblasen zu entfernen und die Proben auf Goldelektroden (Durchmesser 11 mm, 100 $\mu$l) oder auf ein Deckglas (Durchmesser 8 mm, 67 $\mu$l) pipettiert und mit einem 11 mm oder 8 mm großen hydrophoben (Sigmacote® behandelten) Deckglas abgedeckt. Die Vernetzung des Hydrogels erfolgt über die Reaktion der Thiol- und Maleimidgruppen (Michael-Additionsreaktion). Die Polymerisation erfolgt für mindestens 30 min bei Raumtemperatur in einer feuchten Kammer, um ein Austrocknen der Gele zu vermeiden. Für die Kryogelierung werden die Proben über Nacht bei -15 °C polymerisiert. Anschließend werden die vollständig polymerisierten Hydrogele über Nacht in 1 x PBS (0,154mmol/l NaCl, pH 7,4) gequollen. Der Feststoffanteil der Gele beträgt ca. 3 % (m/v).

## Maleimide-Thiol Drei-Komponenten-System zur Herstellung eines Polymernetzwerks als Vorstufe des erfindungsgemäßen Hydrogelmaterials

Für eine präzise Einstellung der integralen negativen Ladung im Hydrogelmaterial wird ein drei-Komponenten System verwendet. Es werden starPEG-Thiol, starPEG-Maleimide und Maleimid-funktionalisierte anionisch geladene Hydrogelbausteine Polymere in 0,01 x PBS in einer vorgegebenen Konzentration gelöst und in Abhängigkeit der gewünschten integralen Ladung in verschiedenen Verhältnissen gemischt. Die Mischungsverhältnisse sind Tabelle 2 zu entnehmen. Durch Zugabe von 0,01 M HCl beziehungsweise 0,01 M NaOH wird die Zeit zur Gelierung auf weniger als 5 min eingestellt. Anschließend werden die Lösungen gemischt und der Probenkörper analog zum Zwei-Komponenten-System bei Raumtemperatur (30 min) beziehungsweise 15 °C (über Nacht) erzeugt. Die vollständig polymerisierten Hydrogele werden über Nacht in 1 x PBS (pH 7) gequollen. Der Feststoffanteil der Gele beträgt ca. 3 % (m/v).

Die Eigenschaften der ungeladenen und anionisch geladenen Bausteine sind in Tabelle1 gezeigt.

## EDC/NHS-System zur Herstellung eines Polymernetzwerks als Vorstufe des erfindungsgemäßen Hydrogelmaterials

Für das EDC/NHS-System werden für ein molares Verhältnis von UGB3 sternPEG-Amin zu nicht-maleimidierten GB1 von 2 0,167 mg/$\mu$l von UGB3, 0,145 mg/$\mu$l GB1, 0,1 mg/$\mu$l EDC (N-(3-Dimethylaminopropyl)-N'-ethylcarbodiimid,) und 0,1 mg/$\mu$l NHS (N-Hydroxysulfosuccinimid) in eiskaltem MilliQ-Wasser gelöst. Für 1 ml finales Gelvolumen werden zunächst 85,20 $\mu$l EDC-Lösung und 48,25 $\mu$l NHS-Lösung zu 533,22 $\mu$l Heparin-Lösung pipettiert, in einem Labormixer gemischt (Vortexen) und für 15 min auf Eis inkubiert. Anschließend erfolgt die Zugabe von 333,33 $\mu$l starPEG-Lösung und das erneute Mischen durch Vortexen. Direkt im Anschluss erfolgt das Erzeugen der gewünschten Formkörper identisch zum Zwei-Komponenten-System. Die Polymerisation erfolgt über Nacht bei Raumtemperatur oder -15 °C in einer feuchten Kammer um das Austrocknen der Hydrogele zu vermeiden. Der Gesamtfeststoffgehalt der Gele beträgt ungefähr 10 % bei einer finalen starPEG-Konzentration von 0,00784 mol/l und einer finalen Heparin Konzentration von 0,00392 mol/l. Die vollständig polymerisierten Hydrogele wurden über Nacht in 1 x PBS (pH 7) gequollen.

## Synthese des elektrisch leitfähigen Hydrogelmaterials als pseudo-interpenetrierendes Netzwerk

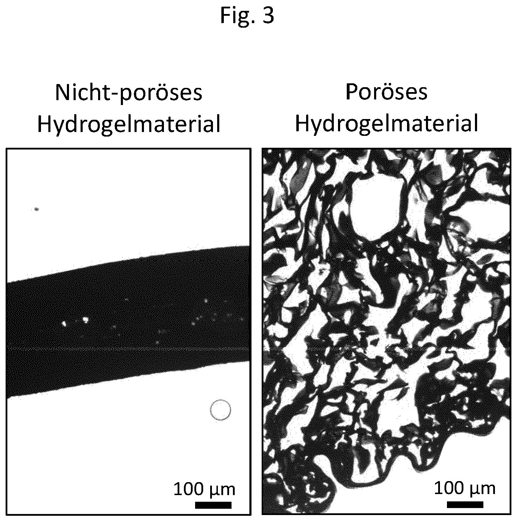

Die Synthese des elektrisch leitfähigen pseudo-interpenetrierenden Polymernetzwerks ist für sämtliche Hydrogelmaterialien der zuvor beschriebenen Bildungsvorschriften möglich. Für die Synthese des elektrisch leitfähigen pseudo-interpenetrierenden Poly-3,4-ethylendioxythiophen (PEDOT)-Netzwerks mit PEDOT als elektrisch leitfähige Komponente, werden die vollständig gequollenen anionisch geladenen Polymernetzwerke im ersten Schritt für 3 Stunden in einer 0,4 M Ammoniumperoxodisulfat (APS) Lösung in 1 M HCl bei Raumtemperatur inkubiert. Anschließend wird das Hydrogel für 6 Stunden in eine 0,4 M 3,4-Ethylendioxythiophen (EDOT) Lösung in Mineralöl in einem Rotationsschüttler inkubiert. In dieser Zeit erfolgt die oxidative Polymerisation des EDOT zu einem elektrisch leitfähigen PEDOT-Netzwerk, welches mit zunehmender Polymerisation des PEDOT eine schwarze Farbe annimmt (Figur 3). Anschließend werden die so hergestellten Hydrogelmaterialien über Nacht in Mineralöl gewaschen, um nicht reagierte Monomere zu entfernen. Zum Entfernen des Mineralöls werden die Hydrogelmaterialien anschließend in Hexan gewaschen. Der Waschvorgang kann wiederholt werden. Anschließend werden die Hydrogelmaterialen für mindestens 24 h in PBS (pH 7,4) gewaschen.

Alle Hydrogelmaterialtypen sind in **Tabelle 2** zusammengefasst. Bei den angegebenen Konzentrationen handelt es sich um die Konzentration in der fertigen Gelmischung. Die Hydrogelmaterialtypen GB1-UGB2 16, GB1-UGB2 18, GB2-UGB2-UGB1 19, GB2-UGB2-UGB1 20, GB3-UGB2-UGB1 21 und GB4-UGB2-UGB1 22 sind Hydrogele, welche nicht mit PEDOT funktionalisiert wurden. Hydrogelmaterialtyp UGB1-UGB2 15 ist ein reines PEG-PEG Hydrogel, welches keine anionischen Ladungen trägt, allerdings PEDOT funktionalisiert ist.

## Elektrische Charakterisierung

Die elektrische Charakterisierung sämtlicher Hydrogele erfolgte mittels Impedanzspektroskopie beziehungsweise zyklischer Voltammetrie in einem 3-Elektroden-Setup in 100 ml 1x PBS (pH 7). Für Beide Messmethoden wurden 100 $\mu$l Gel-Probenkörper (Volumen ungequollen) auf einer Gold-Netzelektrode hergestellt, welche als Arbeitselektrode dient. Als Gegenelektrode wurde eine poröse Kohlenstoff-Elektrode (BioLogic A-010530) mit deutlich größerer Oberfläche verglichen zur Arbeitselektrode verwendet. Als ReferenzElektrode diente eine Ag/AgCl-Elektrode (Metrohm, Part No. 6.0726.100). Als Messgerät diente ein Potentiostat (Metrohm Autolab PGSTAT204 oder PalmSens 4).

## Impedanzspektroskopie

Für die Impedanzspektroskopie wird ein Effektivpotential von 10 mV$_{rms}$ angelegt und die Impedanz sowie die der Phasenwinkel in einem Frequenzbereich von 0,01 Hz bis $10^5$ Hz mit 10 Messpunkten je Dekade gemessen. Die Impedanz der verschiedenen Hydrogelmaterialtypen wurde bei einer Frequenz von 0,01 Hz verglichen. Die elektrische Leitfähigkeit verhält sich umgekehrt proportional zur Impedanz. Steigt die Impedanz so sinkt die elektrische Leitfähigkeit.

## Zyklische Voltammetrie

Die Berechnung der Ladungsspeicherkapazität erfolgt anhand der Messung der zyklischen Voltammetrie. Dafür wird unter Verwendung des oben genannten 3 Elektroden-Aufbaus der Stromfluss zwischen Arbeitselektrode (Hydrogel) und Gegenelektrode (Kohlenstoff-Elektrode) gemessen, während in 5 zyklischen Durchläufen das angelegte elektrische Potential von -0,6 bis 0,8 V (Potential zwischen Arbeitselektrode und Ag/AgCl-Referenzelektrode) variiert wird. Die Scan-Rate beträgt 50 mV/s. Anschließend wird der negative Teil der Fläche unter der Kurve integriert (MultiTrace 4.3, PalmSens 4) und daraus die Ladungsspeicherkapazität des Hydrogels mit nachfolgender Formel berechnet:

$$Ladungsspeicherkapazit\text{ä}t = \frac{I * U}{Scan - Rate}$$

Als Integral gibt die Software den Wert I * U in der Einheit [A] * [V] aus. Dabei gilt [A] = [C/s]. Durch die Division der Scan-Rate [V/s] resultiert die Ladung [C], welche durch das Material übertragen wird. Diese wird anschließend in das Verhältnis zum Volumen gesetzt, da die Grenzfläche zum umgebenden Medium im kompletten Volumen des Hydrogels vorhanden ist und sich somit keine Oberfläche berechnen lässt. Die Angabe der Werte erfolgt in Ladung pro Milliliter Hydrogel.

## Elektrische Eigenschaften der Hydrogelmaterialien

Direkt nach der oxidativen Polymerisation besitzt PEDOT eine inhärente positive Ladung (10.1021/acs.jpcb.9b01745, 10.1021/acsapm.8b00061). Durch die nicht-kovalenten Wechselwirkungen zwischen PEDOT und dem anionisch geladenen Polymernetzwerk (Hydrogel) sowie der nicht-kovalenten Wechselwirkung zwischen den einzelnen PEDOT-Polymerketten bildet sich ein pseudo-interpenetrierendes Netzwerk zwischen dem anionisch geladenen Polymernetzwerk und PEDOT aus. Gleichzeitig fungieren die negativen Ladungen des anionisch geladenen Polymernetzwerks als p-Dotierung für PEDOT. In Abhängigkeit der integralen und lokalen Ladungsdichte (Parameter P0/P1 und P2) ändert sich der Dotierungsgrad des PEDOT. Dies hat einen direkten Einfluss auf die elektrischen Eigenschaften (Impedanz und Ladungsspeicherkapazität) des elektrisch leitfähigen Hydrogelmaterials. Bei gleichem P2 und P3 wurden über einen P1-Bereich von 110 bis 2 $\mu$mol/ml eine Ladungsspeicherkapazität von 3040 bis 1505 mC/ml und eine Impedanz von 30 bis 93 $\Omega$ für die Hydrogelmaterialtypen GB1-UGB2 01 und GB1-UGB2-UGB1 02-04 und in einem P1-Bereich von 128 bis 2 $\mu$mol/ml eine Ladungsspeicherkapazität von 3040 bis 1894 mC/ml und eine Impedanz von 36 bis 74 $\Omega$ für die Hydrogelmaterialtypen GB2-UGB2 05 und GB2-UGB2-UGB1 06-08 (Tabelle 3-1 und 3-2) erhalten. Da es sich bei PEDOT um ein hydrophobes Polymer handelt, ist die Verteilung und die Vernetzung der PEDOT-Ketten untereinander stark von der Hydrophobizität der Umgebung und damit der Amphiphilie des anionisch geladenen Polymernetzwerks (Parameter P3) abhängig, was ebenfalls einen Einfluss auf die elektrischen Eigenschaften des elektrisch leitfähigen Hydrogelmaterials hat. In den Ausführungsbeispielen zeigt sich, dass bei einem hohen Wert des Parameters P3 von 6,8 *10$^{-3}$ 1/A$^2$ (GB2-UGB2-UGB1 08), siehe Tabelle 2 im Vergleich zu einem niedrigen Wert für P3 von -5,9*10$^{-3}$ 1/A$^2$ (GB1-UGB2-UGB1 04), bei gleich bleibendem Wert für P2 (4,5 mmol/(g/mol)) und P1 (2 $\mu$mol/ml) die höhere Hydrophobizität zu einer höheren Ladungsspeicherkapazität (1894 vs. 1505 mC/ml) und geringerer Impedanz (74 $\Omega$ vs. 93 $\Omega$) führt (Tabelle 3-1 und 3-2). Erhöht man den Abstand zwischen den stark anionisch geladenen Gruppen entlang der Polymerketten (verringerter Wert P2, steht für eine geringere lokale Ladungsdichte) so erhält man bei ähnlichem P3 und annähernd gleichem P1 eine deutlich geringere Ladungsspeicherkapazität bei gestiegener Impedanz. Dieser Unterschied zeigt sich besonders bei einem Vergleich bei niedrigem P1 von 9 mmol/ml, P2 von 0,9 mmol/(g/mol) und P3 von 0,7*10$^{-3}$ 1/A$^2$ (GB4-UGB2-UGB1 12) im Vergleich zu einem P1 von 11 beziehungsweise 4 mmol/ml, P2 von 4,5 mmol/(g/mol) und P3 von -5,9*10$^{-3}$ 1/A$^2$ (GB1-UGB2-UGB1 02/03). Trotz des geringeren Wertes für P3 und einem annähernd gleichen beziehungsweise geringerem Wert für P1 besitzen die Hydrogele mit höherem P2 (GB1-UGB2-UGB1 02/03) eine deutlich höhere Ladungsspeicherkapazität (2350 und 1811 mC/ml und geringere Impedanz (42 und 81 $\Omega$) im Vergleich zu GB4-UGB2-UGB1 12 (910 mC/ml und 150 $\Omega$). Trotz der moderaten Werte für P1 und P3 verhält sich GB4-UGB2-UGB1 12 wie ein Hydrogel ohne anionisch geladene Gruppen nach PEDOT Funktionalisierung, siehe UGB1-UGB2 15 (reines PEG-Hydrogelmaterial, 921 mC/ml und 167 $\Omega$). Es lassen sich somit durch die Veränderungen im Dotierungsgrad (P1, P2) sowie der Hydrophobizität (P3) leitfähige Hydrogele mit unterschiedlichen elektrischen Eigenschaften erhalten. Durch eine hohe integrale Anzahl an anionischen Gruppen (d.h. einem hohen Wert für P0 bzw. P1) sowie einen geringen Abstand zwischen den stark anionisch geladenen Gruppen (P2) können Hydrogele mit hoher Leitfähigkeit erhalten werden. P0 scheint einen ähnlichen Einfluss wie P1 auf die elektrischen Eigenschaften zu haben, der vermutlich auf den Anteil der stark anionischen Gruppen zurückzuführen ist. Die schwach anionischen Gruppen, hier beispielsweise Carboxylgruppen mit einem intrinsischen pKa im Bereich von 3,5 bis 4,5 spielen vermutlich eine untergeordnete Rolle bei der Dotierung des PEDOT. Durch den Parameter P3 lässt sich ebenfalls die Interaktion zwischen hydrophilen/amphiphilen anionischen Polymer und hydrophoben elektrisch leitfähigem Polymer (PEDOT) konfigurieren. Durch eine erhöhte Hydrophobizität der anionisch geladenen Hydrogelbausteine (hoher Wert für Parameter P3) lassen sich bereits bei deutlich geringerem P1 gesteigerte Leitfähigkeiten (d.h. geringere Impedanzen) erzeugen. Dies liegt an der hohen Affinität der hydrophoben PEDOT-Einheiten zu den hydrophoben Gruppen auf den anionischen geladenen Hydrogelbausteinen, welche möglicherweise die Dotierung positiv beeinflussen. Zusätzlich erleichtern die hydrophoben Gruppen bereits während der PEDOT Synthese die Penetration und Verteilung der Monomereinheiten (EDOT) im anionisch geladenen Polymernetzwerk. Die elektrisch leitfähigen Hydrogelmaterialien mit sehr hoher Leitfähigkeit (geringer Impedanz) und hoher Ladungsspeicherkapazität können als biokompatible Elektrodematerialien zur Stimulation von Zellen oder Gewebe verwendet werden. Aufgrund der hohen Hydratisierung und Weichheit dieser Hydrogele, welche biologischem Gewebe sehr ähnlich sind, können die Fremdkörperreaktionen des Organismus gegenüber solchen auf den erfindungsgemäßen, elektrisch leitfähigen Hydrogelmaterialien basierenden Elektroden im Vergleich zu klassischen Metallelektroden deutlich reduziert werden. Zusätzlich findet bei klassischen Metallelektroden der Ladungstransfer für die elektrische Stimulation nur an der Kontaktfläche vom Metall zum Gewebe (physiologische Lösung) statt. Aufgrund der Verteilung des leitfähigen Polymers innerhalb des Volumenmaterials und der Möglichkeit, dass Biofluide in das Hydrogel eindiffundieren, kann eine Ladungsübertragung innerhalb des kompletten Volumens stattfinden. Dadurch können für die gleiche Ladungsinjektion geringere Spannungen verwendet werden, was die Wärmeentwicklung und daraus resultierende potentielle Gewebeschäden im Vergleich zu klassischen Metallelektroden reduziert.

### Wirkstoffsequestrierung und -freisetzung

Die Probenvorbereitung für die Wirkstoffsequestrierung und -freisetzung erfolgt identisch zur Charakterisierung der elektrischen Eigenschaften. 100 $\mu$l der verschiedenen anionisch geladenen Polymernetzwerke wurden auf eine Gold-Netzelektrode aufgebracht und mittels nachfolgender Penetration und Polymerisation von EDOT-Monomeren zu PEDOT elektrisch leitfähig gemacht.

### Wirkstoffsequestrierung

Nach gründlichem Waschen der elektrisch leitfähigen Hydrogelmaterialien in PBS erfolgte die Sequestrierung verschiedener Substanzen mit einer Konzentration von jeweils 100 ng/ml Substanz in 2,5 ml PBS mit 0,1% BSA (um die physiologische Situation mit einem Trägerprotein nachzustellen. Das entspricht 250 ng pro Protein und Hydrogelmaterial. Die Sequestrierung erfolgt in einem 5 ml *low binding* Eppendorf Tube (Eppendorf) um unspezifische Bindung der Proteine zum Reaktionsgefäß zu minimieren. Die Aufnahme erfolgt über 24 h. Die Sequestrierung erfolgt bei 500 mV, 0 mV (passiv) und -500 mV. Die aktive Sequestrierung erfolgt in einem 3-Elektroden-Setup ähnlich der elektrischen Charakterisierung. Als Arbeitselektrode diente das leitfähige Hydrogel auf der Gold-Elektrode. Diese befand sich zusammen mit der Referenzelektrode, einem Ag/AgCl Draht, in einem 5 ml *low binding* Eppendorf Tube. Die Gegenelektrode, eine poröse Kohlenstoffelektrode (BioLogic A-010530) mit deutlich größerer Oberfläche verglichen zur Arbeitselektrode, befand sich in einem separaten Gefäß mit 100 ml PBS. Geschlossen wurde der Stromkreis durch eine Salzbrücke bestehend aus einem PVC-Schlauch, welcher mit einem 25%igen Polyacrylamidhydrogel (nach Herstellerangaben angeben) gequollen in PBS gefüllt war. Im Eppendorf-Gefäß war der Schlauch zusätzlich mit einer 1000 Da Dialysemembran verschlossen um ein Eindringen der Substanzen zu verhindern. Für das Anlegen eines definierten Potentials diente ein Potentiostat (Metrohm Autolab PGSTAT204 oder PalmSens 4). Als Proben wurden 100 $\mu$l der Lösung vor und nach der Sequestrierung entnommen. Nach Bestimmung der Konzentrationen der verschiedenen Proteine nach Herstellerangaben unter Verwendung des Multiplex Assays Kits (Luminex Technology, ThermoFisher) wurde die aufgenommene Menge an Protein prozentual berechnet.

Für die Sequestrierung von Substanzen spielen vorwiegend die integrale P0 beziehungsweise P1 und lokale Ladungsdichte (P2) bei den verschiedenen angelegten Spannungen im gesamten Hydrogelmaterial (anionisch geladenes Polymernetzwerk -PEDOT pseudo IPN) eine Rolle. Die höchste Sequestrierung konnte dabei unabhängig von der Ladung des gebundenen Moleküls ohne angelegtes Potential gemessen werden (Tabelle 4-1, 4-2, 4-3 und 4-4). Im Vergleich zu nicht leitfähigen Hydrogelen (ohne PEDOT) mit ähnlichen P1 und P2 zeigt sich, dass die elektrisch leitfähigen Hydrogelmaterialien eine geringere Aufnahme positiv geladener Substanzen (GB2-UGB2-UGB1 09; 72,3 % SDF-1$\alpha$; 72,7 % FGF-2; 70,8 % IL-8 gegenüber GB2-UGB2-UGB1 19; 98,2 % SDF-1$\alpha$; 85,0 % FGF-2; 96,7 % IL-8) aufweisen (siehe Tabelle 4-1, 4-2, 4-3 und 4-4). Bei negativ geladenen Molekülen zeigt sich ein umgekehrter Effekt. Eine Aufnahme von 49,9 % GM-CSF und 37,1 % EGF bei GB2-UGB2-UGB1 09 im Vergleich zu 43,3 % GM-CSF und 33,5 % EGF bei GB2-UGB2-UGB1 19 konnten gemessen werden. Dieser Effekt ist noch stärker ausgeprägt bei einem geringerem P1. So konnten 77,8 % GM-CSF und 69,2 % EGF bei GB2-UGB2-UGB1 10 im Vergleich zu 0,0 % GM-CSF und 21,0 % EGF bei GB2-UGB2-UGB1 20 gebunden werden. Grund dafür ist wahrscheinlich die positive Ladung des PEDOT. PEDOT weist ohne Anlegen eines elektrischen Potentials eine positive Ladung von ca. 33 % der Monomereinheiten auf, welche mit negativ geladen Substanzen eine Wechselwirkung verursachen und diese wahrscheinlich binden können (10.1021/acsapm.8b00061, 10.1021/acsami.5b04768). Zusätzlich zeigt sich, dass bei einem niedrigen P1 auch positiv geladene Substanzen durch die elektrisch leitfähigen Hydrogele besser im Vergleich zur nicht leitfähigen Kontrolle gebunden werden konnten. So konnten bei GB2-UGB2-UGB1 10, 76,9 % SDF-1$\alpha$; 86,1 % FGF-2; 39,9 % IL-8, gegenüber GB2-UGB2-UGB1 20, 60,5 % SDF-1$\alpha$; 41,6 % FGF-2; 17,1 % IL-8, gebunden werden. Dies lässt vermuten, dass auch für die Bindung vorwiegend positiv geladener Substanzen das leitfähige Polymer PEDOT vor allem durch wechselseitige Ladungskompensation mit den anionisch geladenen Gruppen des Hydrogelmaterials eine wichtige Rolle spielt. Sowohl die ionische Bindung negativ geladener Domänen im Protein durch PEDOT als auch hydrophobe Wechselwirkungen zwischen Protein und dem PEDOT spielen hier eine wichtige Rolle.

Wird ein positives Potential von 500 mV angelegt, verringert sich insgesamt die Sequestrierung von Substanzen (Tabelle 4-1, 4-2, 4-3 und 4-4). Die ist vor allem bei positiv geladenen Substanzen zu beobachten. Bei negativ geladenen Substanzen, wie beispielsweise GM-CSF und EGF, lässt sich hingegen eine geringe Steigerung beziehungsweise eine nahezu identische Sequestrierung beobachten. So konnten 49,9 % GM-CSF und 37,1 % EGF vor Anlegen des Potentials und 59,2 % GM-CSF und 67,0 % EGF nach Anlegen des Potentials bei GB2-UGB2-UGB1 09 beziehungsweise 77,8 % GM-CSF und 69,2 % EGF vor Anlegen des Potentials und 78,4 % GM-CSF und 64,8 % EGF nach Anlegen des Potentials bei GB2-UGB2-UGB1 09 gebunden werden. Grund dafür kann sein, dass sich durch das Anlegen eines positiven Potentials die Ladung des PEDOT erhöht. (10.1021/acsapm.8b00061, 10.1021/acsami.5b04768). Dies führt zu einer positiven Beeinflussung der Bindung negativ geladener Proteine wie GM-CSF und EGF durch die erhöhte ionische Wechselwirkung. Da die negative Ladung der anionischen Komponente im Hydrogel hingegen konstant ist und die stärkere positive Ladung des PEDOT zur teilweisen Ladungskompensation führen kann, verringert sich summarisch die Aufnahme positiv geladener Proteine.

Wird ein negatives Potential angelegt, kann die positive Ladung des PEDOT reduziert beziehungsweise sogar komplett neutralisiert werden (10.1021/acsapm.8b00061, 10.1021/acsami.5b04768). Bei einem Potential von -500 mV zeigt sich vorwiegend eine Reduzierung der Substanz-Sequestrierung unabhängig von der Ladung der Substanzen und unabhängig vom P1 und P2 der Hydrogelmaterialien (Tabelle 5-1, 5-2, 5-3 und 5-4). So sinkt die Sequestrierung von SDF-1$\alpha$ von 72,3 % auf 47,2 %, NGF-$\beta$ von 94,1 % auf 75,0 % und IL-8 von 70,8 % auf 35,4 % in GB2-UGB2-UGB1 09. Ein gleicher Trend lässt sich für GB2-UGB2-UGB1 10 und GB3-UGB2-UGB1 11/12 beobachten. Dies bestätigt die Vermutung, dass für die Bindung vorwiegend positiv geladener Substanzen die positive Ladung des PEDOT eine wichtige Rolle spielt. Dies kann z.B. durch die Bindung negativ geladener Domänen der Substanzen (Proteine) an die positiven Ladungen von PEDOT, das zuvor beschriebene Phänom, verursacht werden. Bei der Sequestrierung negativ geladener Substanzen zeigt sich bei -500 mV eine sehr starke Reduzierung der Sequestrierung. So sinkt die Sequestrierung von GM-CSF von 49,9 % auf 17,9 % und EGF von 37,1 % auf 0,0 % in GB2-UGB2-UGB1 09 beziehungsweise von GM-CSF von 77,8 % auf 44,9 % und EGF von 69,2 % auf 42,8 % in GB2-UGB2-UGB1 10. Der Grund für diese starke Verringerung der Sequestrierung von negativ geladenen Substanzen bei negativem Potential liegt vermutlich in der Reduzierung der positiven Ladung des PEDOT. Dadurch finden sich deutlich weniger Bindungsstellen für negativ geladene Substanzen.

## Wirkstofffreisetzung

Nach der Sequestrierung (Beladung der Hydrogele nach der zuvor angesprochen dritten Beladungsmethode) erfolgte die elektrisch gesteuerte Freisetzung der bioaktiven Moleküle. Aufgrund der im Durchschnitt höchsten Sequestrierung wurden dafür die Proben verwendet, welche bei einem Potential von 0 mV (passiv) für 24 h mit Substanzen beladen wurden. Eine kontrollierte Freisetzung von aktiv sequestrierten Substanzen kann ebenfalls nach dem im folgenden beschriebenen Protokoll durchgeführt werden.

Nach der Beladung der Hydrogele erfolgte zunächst ein kurzer Waschschritt um schwach gebundene Proteine zu entfernen. Dafür wird das Hydrogel zunächst bei 3000 rpm für 1 min zentrifugiert um anhaftende Flüssigkeit zu entfernen. Anschließend werden die Hydrogele in 1 ml PBS mit BSA gewaschen und erneut bei 3000 rpm für 1 min zentrifugiert. Die Freisetzung erfolgt ebenfalls in dem im Kapitel Wirkstoffsequestrierung beschriebenen 3-Elektroden-Setup bei einem angelegten Potential von 500 mV, 0 mV und -500 mV. Die Probenentnahme erfolgte für die Ausgangslösung (Kontrolle), nach Sequestrierung, nach dem Waschen und für die Freisetzung nach 0 min, 10 min, 30 min, 1 h, 6 h, 8 h und 24 h. Da sich bereits nach 8 h eine Sättigung der freigesetzten Proteine beobachtet werden konnte, sind die Ergebnisse der im Plateau liegenden Freisetzung nach 8 h gezeigt (Tabelle 5-1, 5-2, 5-3 und 5-4). Die Bestimmung der Konzentration der verschiedenen Substanzen erfolgte nach Herstellerangaben unter Verwendung des Multiplex Assays Kits (Luminex Technology, ThermoFisher).

Die Freisetzung der gebundenen Substanzen lässt sich in Abhängigkeit der konfigurierten Parameter des anionisch geladenen Polymernetzwerks in Kombination mit der Ladung des PEDOT einstellen (Tabelle 5-1, 5-2, 5-3 und 5-4). Bei einem hohem P1 (60 $\mu$mol/ml, GB2-UGB2-UGB1 09) erfolgt lediglich eine sehr geringe Freisetzung von positiv geladenen Substanzen (0,0 % SDF-1$\alpha$; 0,0 % FGF-2; 0,5 % IL-8). Für positiv geladene Substanzen kann hingegen eine moderat geringe Freisetzung beobachtet werden (2,3 % GM-CSF; 9,1 % EGF). Durch die wahrscheinliche Überkompensation der positiven Ladung des PEDOT durch die anionischen Gruppen, kann eine Freigabe von negativ geladenen Substanzen wahrscheinlich deutlich schneller erfolgen als die Freigabe von positiv geladenen Proteinen. Bei niedrigem P1 (2 $\mu$mol/ml, GB2-UGB2-UGB1 10) erfolgt eine gesteigerte Freisetzung der positiv geladenen Substanzen (0,8 % SDF-1$\alpha$; 0,3 % FGF-2; 11,7 % IL-8). Negativ geladene Substanzen hingegen werden weniger freigesetzt verglichen mit einem höheren P1 (0,4 % GM-CSF; 2,0 % EGF). Verringert man P2 bei ähnlichen P1 Werten (GB3-UGB2-UGB1 11/12) so kann eine gesteigerte Freisetzung negativ geladener Substanzen (4,3/3,7 % GM-CSF; 94,1/73,5% EGF) bei nahezu unveränderter Freisetzung von positiv geladenen Substanzen erreicht werden (1,6/0,2% SDF-1α; 0,0/0,0% FGF-2; 1,4/1,2% IL-8) werden (Figur 8 Tabelle 5). Grund dafür ist ebenfalls die Kompensation positiver Ladungen im PEDOT durch anionische Gruppen trotz der geringeren Ladungsdichte.

Legt man ein Potential von 500 mV an, so kann ein Zurückhalten gebundener Substanzen unabhängig von deren Ladungen beobachtet werden. Der Effekt zeigt sich besonders deutlich für GB2-UGB2-UGB1 09. Hier konnte die Freisetzung von nahezu allen 14 Proteinen auf 0 % reduziert werden (Tabelle 5-1, 5-2, 5-3 und 5-4). Auch bei moderatem P1 (9 μmol/ml) und sehr geringem P2 (0,94 mmol/(g/ml)) in GB3-UGB2-UGB1 12 konnte die Freisetzung sämtlicher Substanzen auf nahezu 0 % gesenkt werden (Tabelle 5-1, 5-2, 5-3 und 5-4). Grund dafür könnte in der Quellrate der Hydrogele liegen. Bei einem positiven Potential kompensiert die erhöhte positive Ladung des PEDOT mehr anionisch geladene Gruppen. Geht man davon aus, dass mehr anionische Gruppen verglichen zu den positiven Ladungen des PEDOT im Hydrogelmaterial vorhanden sind, verschiebt sich die gesamte Ladung des Hydrogels hin zum neutralen, wodurch es zu einem Wasserverlust des Hydrogels und dadurch zu einem Schrumpfen kommt. Dies führt zu einer verringerten Maschenweite der Polymerketten, was wiederum eine reduzierte Freisetzung der Proteine unabhängig von deren Ladung zur Folge hat. Wird ein Potential von -500 mV angelegt, zeigt sich eine gesteigerte Freisetzung bei einem Großteil der Substanzen (Tabelle 5-1, 5-2, 5-3 und 5-4). 5). Dies betrifft vorwiegend negativ geladene bioaktive Moleküle. So steigt nach Anlegen des Potentials die Freisetzung von TNF-α, GM-CSF und EGF für den Hydrogelmaterialtyp GB2-UGB2-UGB1 09 von 1,4/2,3/9,2 % auf 5,3/10,4/67,2 % und GB2-UGB2-UGB1 10 von 1,7/0,4/2,0 % auf 53,2/5,9/72,6 %. Dieser Trend kann auch für die Hydrogelmaterialtypen GB3-UGB2-UGB1 11/12 beobachtet werden. Interessant ist dabei, dass Substanzen die bereits ohne angelegtes Potential eine größere Freisetzung aufweisen durch Anlegen des negativen Potentials am meisten freigesetzt werden. Grund dafür ist höchstwahrscheinlich die Verringerung/Neutralisierung der positiven Ladung des PEDOT. Dadurch sind mehr freie anionische Gruppen vorhanden, die durch ionische Wechselwirkungen eine Abstoßung negativ geladener Proteine bewirken. Allerdings kann auch bei positiv geladenen Substanzen eine gesteigerte Freisetzung beobachtet werden. So steigert sich die Freisetzung von FGF-2, IL4 und IL8 für den Hydrogelmaterialtyp GB2-UGB2-UGB1 10 von 0,3 % auf 2,6 %, 0,6 % auf 1,7 % und 11,7 % auf 26,3 %. Grund hierfür könnte ebenfalls die gesteigerte Anzahl an freien anionisch geladenen Gruppen durch fehlende Ladungskompensation mit PEDOT sein. Die dadurch bewirkte erhöhte negative Nettoladung des Hydrogels führt zu einem Quellen. Dadurch wird eine Freisetzung unabhängig von der Ladung begünstigt, wodurch auch positiv geladene Substanzen im höheren Maße freigesetzt werden können.

## Hydrogel Biokompatibilität

Für die Untersuchung der Biokompatibilität wurde die PC12-Zelllinie und der Hydrogelmaterialtyp GB1-UGB2 17 verwendet. Bei der Zelllinie handelt es sich um Phäochromozytom-Zellen, welche durch Zugabe von NGF-β innerhalb von wenigen Tagen zu Neuron-artigen Zellen differenziert werden können. Die PC-12-Zelllinie ist ein weit verbreitetes Modell für neuronale Differenzierung. Für die Kultivierung mit PC12 Zellen wurden die gewaschenen und in PBS gequollenen elektrisch leitfähigen Hydrogele zunächst für 10 min in 70 % Ethanol gespült um eventuelle Mikroorganismen abzutöten und anschließend in sterilem PBS gewaschen. Nach der Behandlung mit Collagen Typ I (50 μg/ml, 1 ml/100 μl Gel, Gibco™) in 20 mM steriler Essigsäure für 2 h bei Raumtemperatur wurden die Gele für 30 min in PBS gewaschen. Für NGF-β beladene Gele erfolgte eine zusätzliche Inkubation mit NGF-β (100 ng/ml, Sigma-Aldrich) in PBS mit 1 % BSA über Nacht. Anschließend wurden 50.000 Zellen pro cm² Geloberfläche ausgesät. Die Aussaat der Zellen sowie deren Inkubation erfolgte in RPMI1640 (Gibco™) mit einem Zusatz von 10 % Pferdeserum (Gibco™), 5 % fetalem Kälberserum (Gibco™) und 1 % Penicillin und Streptamycin (Gibco™) im Medium. Je nach Kultivierungsbedingung erfolgte die Verwendung des puren Mediums, die Zugabe von 100 ng/ml NGF-β in das Medium oder die Verwendung von nährstoffreduziertem Medium (reduziert) mit nur 1 % Pferdeserum (Gibco™) und 0,5 % fetalem Kälberserum. Nach einer Inkubation von 5 beziehungsweise 7 Tagen mit einem Mediumwechsel aller 2 Tage erfolgte das Fixieren der Zellen für 20 min bei Raumtemperatur in 4 % PFA in PBS. Für die Bildgebung wurden die Zellen mit Phalloidin (Abcam) und DAPI (Pierce) nach Herstellerangaben gefärbt. Bilder wurden mit einem Fluoreszenzmikroskop aufgenommen. Die Auswertung der Zellzahl und des Zellumfangs erfolgte mit FIJI/ImageJ unter der Verwendung des *watershed* und *analyze particles* (Zellzahl) und des *skeletonize* Plugins.

Für alle Kultivierungsbedingungen wurden Zelladhäsion und Zellproliferation auf den Gelen beobachtet. Im Vergleich von 7 zu 5 Tagen erfolgte eine starke Zunahme der Zellzahl bei Abnahme des Zellumfangs auf den Hydrogelen ohne Zusatz von NGF-β **(Tabelle 6).** Dies spricht für eine hohe Zellproliferation. Die Zugabe von NGF-β in das Medium führt zur Differenzierung der PC12-Zellen nach bereits 5 Tagen (Tabelle 6). Dies konnte in einer Zunahme des Zellumfangs durch das Wachstum axonartiger Strukturen bestätigt werden. Eine Zugabe von NGF-β in das Hydrogel nicht aber ins Medium führt zu einer nur geringen Steigerung des Zellumfangs bei nur gering steigender Zellzahl ( Tabelle 6). Ohne Stimulus ist die Menge an freigesetztem NGF-β zu gering um eine vollständige Differenzierung der Zellen zu erreichen. Dies liegt an der hohen Affinität von NGF-β zum Hydrogel. Reduziert man den Nährstoffgehalt im Medium so lässt sich eine gesteigerte Differenzierung erreichen. Die Zellen besitzen demnach in allen Kultivierungsbedingungen die Fähigkeit auf den Hydrogelen zu proliferieren und differenzieren, was eine hohe Biokompatibilität der Hydrogele bestätigt.

**Bezugszeichenliste**

| | |
|---|---|
| 1 | elektrisch leitfähiges Hydrogelmaterial / Hydrogelmaterial |
| 2 | Polymernetzwerk |
| 3 | anionisch geladene Bausteine |
| 4 | Vernetzermolekül |
| 5 | elektrisch leitfähige Komponente / PEDOT |
| 6 | bioaktive Substanz / bioaktive Substanzen / Signalprotein |
| 7 | Spannungsquelle |

**Tabelle 1**

EP 4 251 221 B1

Tabelle 1: Übersicht über die geladenen und ungeladenen Hydrogelbausteine der Polymernetzwerke

| Geladene und ungeladene Bausteine | Bau steine Abkürzung | M [g/mol] | Anzahl der Maleimidgruppen [1/mol] | Anzahl der anionischen Gruppen [1/mol] | Anzahl der anionischen Gruppen (pKa<2.5, [1/mol]) | P2 [mmol/ (g/mol)] | P3 *10⁻³ [1/Å²] |
|---|---|---|---|---|---|---|---|
| Heparin | GB1 | 14000 | 7 | 87,6 | 67,6 | 4,5 | -5,9 |
| Poly(4-Styrensulfonsäure-co-Maleinsäure) 3:1 | GB2 | 21100 | 9 | 142,8 | 92 | 4,5 | 6,8 |
| Poly(Acrylsäure-co-Acrylamidoethan-hydrogensulfat) 1:1 | GB3 | 26200 | 12 | 149 | 80 | 3,2 | -2,5 |
| Poly(Acrylsäure-co-Acrylamidoethanhydrogensulfat) 9:1 | GB4 | 18400 | 12 | 149 | 16 | 0,9 | 0,7 |
| 6-O,N-desulfatiertes Heparin | GB5 | 12300 | 7 | 42,5 | 22,5 | 1,8 | -6,8 |
| 4-Arm-Polyethylenglykol, maleimidterminiert | UGB1 | 10000 | 0 | 0 | 0 | 0 | -2,82 |
| 4-Arm-Polyethylenglykol, thiolterminiert | UGB2 | 10000 | 0 | 0 | 0 | 0 | -2,82 |
| 4-Arm-Polyethylenglykol, amin terminiert thiolterminiert | UGB3 | 10000 | 0 | 0 | 0 | 0 | -2,82 |

Tabelle 2

**Tabelle 2:** Zusammensetzung und Synthese von Polymernetzwerken als Vorstufe eines Hydrogelmaterials

| Hydrogel-materialtyp | geladener Polymer-baustein | Lösungsmittel für geladene und ungeladene Bausteine | GBx (mg/ml) | UGB1 (mg/ml) | UGB2 (mg/ml) |
|---|---|---|---|---|---|
| GB1-UGB2 01 | GB1 | 0,01 x PBS, pH 4 | 30,8 | 0,0 | 21,8 |
| GB1-UGB2-UGB1 02 | GB1 | 0,01 x PBS, pH 4 | 3,7 | 14,3 | 17,3 |
| GB1-UGB2-UGB1 03 | GB1 | 0,01 x PBS, pH 4 | 1,3 | 16,4 | 16,8 |
| GB1-UGB2-UGB1 04 | GB1 | 0,01 x PBS, pH 4 | 0,4 | 17,1 | 16,8 |
| GB2-UGB2 05 | GB2 | 0,01 x PBS, pH 4 | 31,8 | 0,0 | 32,1 |
| GB2-UGB2-UGB1 06 | GB2 | 0,01 x PBS, pH 4 | 4,1 | 14,8 | 25,7 |
| GB2-UGB2-UGB1 07 | GB2 | 0,01 x PBS, pH 4 | 1,4 | 16,6 | 16,8 |
| GB2-UGB2-UGB1 08 | GB2 | GB2: 0,01 PBS, pH 4; UGB: 0,01 PBS, pH 5,9 | 0,4 | 17,1 | 16,8 |
| GB2-UGB2-UGB1 09 | GB2 | 0,01 x PBS, pH 4; | 21,2 | 6,9 | 16,8 |
| GB2-UGB2-UGB1 10 | GB2 | 0,01 x PBS, pH 4; | 0,7 | 17,0 | 16,8 |
| GB3-UGB2-UGB1 11 | GB3 | 0,01 x PBS, pH 4 | 19,8 | 9,5 | 16,8 |
| GB4-UGB2-UGB1 12 | GB4 | 0,01 x PBS, pH 4 | 13,9 | 9,5 | 16,8 |
| GB1-UGB2 13 | GB1 | 0,1 x PBS, pH 6 | 44,8 | 0,0 | 31,7 |
| GB5-UGB2-UGB1 14 | GB5 | 0,01 x PBS, pH 4 | 6,2 | 12,5 | 16,8 |
| UGB1-UGB2 15 | - | UGB1: 0,01 PBS, pH 4; UGB2: 0,01 PBS, pH 6,4 | 0,0 | 17,3 | 16,8 |
| GB1-UGB2 16 | GB1 | 0,1 x PBS, pH 6 | 44,8 | 0,0 | 31,7 |
| GB1-UGB2 17 | GB1 | 0,1 x PBS, pH 6 | 44,8 | 0,0 | 31,7 |
| GB1-UGB2 18 | GB1 | 0,1 x PBS, pH 6 | 44,8 | 0,0 | 31,7 |
| GB2-UGB2-UGB1 19 | GB2 | 0,01 x PBS, pH 4 | 30,8 | 0,0 | 14,9 |
| GB2-UGB2-UGB1 20 | GB2 | 0,01 x PBS, pH 4 | 0,6 | 14,9 | 15,2 |
| GB3-UGB2-UGB1 21 | GB3 | 0,01 x PBS, pH 4 | 29,9 | 0,0 | 22,8 |
| GB4-UGB2-UGB1 22 | GB4 | 0,01 x PBS, pH 4 | 28,9 | 0,0 | 19,6 |

**Tabellen 3-1 und 3-2:** elektrische Eigenschaften der verschiedenen elektrisch leitfähigen Hydrogelmaterialien

Tabelle 3-1

| Hydrogel-materialtyp | GB1-UGB2 01 | GB1-UGB2-UGB1 02 | GB1-UGB2-UGB1 03 | GB1-UGB2-UGB1 04 | GB2-UGB2 05 | GB2-UGB2-UGB1 06 | GB2-UGB2-UGB1 07 | GB2-UGB2-UGB1 08 |
|---|---|---|---|---|---|---|---|---|
| Relativer Quellungsgrad | 1,29 | 1,58 | 1,46 | 1,11 | 1,11 | 1,47 | 1,11 | 1,11 |
| P0 ($\mu$mol/ml) nach Quellung | 46 | 4 | 2 | 1 | 51 | 5 | 2 | 1 |
| P1 ($\mu$mol/ml) nach Quellung | 110 | 11 | 4 | 2 | 128 | 12 | 5 | 2 |
| P2 (mmol/(g/mol)) | 4,5 | 4,5 | 4,5 | 4,5 | 4,5 | 4,5 | 4,5 | 4,5 |
| P3 (1/A-2)*10^3 | -5,9 | -5,9 | -5,9 | -5,9 | 6,8 | 6,8 | 6,8 | 6,8 |
| geladener Polymerbaustei | GB 1 | GB 1 | GB 1 | GB 1 | GB 2 | GB 2 | GB 2 | GB 2 |
| Ladungsspeicherkapazität (mC/ml) | 3040 | 2350 | 1811 | 1505 | 2730 | 3040 | 2140 | 1894 |
| ± S.D. | 28,28 | 14,14 | 21,21 | 128,69 | 14,14 | 56,57 | 28,28 | 82,02 |
| Impedanz bei 0,01 Hz ($\Omega$) | 30 | 42 | 81 | 93 | 42 | 36 | 68 | 74 |
| ± S.D. | 1,52 | 2,12 | 4,06 | 4,65 | 2,08 | 1,82 | 3,39 | 3,70 |

Tabelle 3-2

| Hydrogel-materialtyp | GB2-UGB2-UGB1 09 | G B2-UGB2-UGB1 10 | G B3-UGB2-UGB1 11 | G B4-UGB2-UGB1 12 | GB1-UGB2 13 | GB5-UGB2-UGB1 14 | UGB1-UGB2 15 | GB1-UGB2 16 | GB1-UGB2 17 | Cryogel GB1-UGB2 18 |
|---|---|---|---|---|---|---|---|---|---|---|
| Relativer Quellungsgrad | 1,50 | 1,30 | 1,43 | 1,39 | 1,25 | 1,25 | 1,25 | 1,25 | 1,60 | 1,60 |
| P0 ($\mu$mol/ml) nach Quellung | 24 | 1 | 25 | 26 | 34 | 6 | 0 | 34 | 26 | 26 |
| P1 ($\mu$mol/ml) nach Quellung | 60 | 2 | 43 | 9 | 81 | 9 | 0 | 81 | 81 | 81 |
| P2 (mmol/(g/mol)) | 4,5 | 4,5 | 3,2 | 0,9 | 4,5 | 1,8 | 0,0 | 4,5 | 4,5 | 4,5 |
| P3 (1/A-2)*10^3 | 6,8 | 6,8 | -2,5 | 0,7 | -5,9 | -6,8 | -2,8 | -5,9 | -5,9 | -5,9 |
| geladener Polymerbaustein | GB 2 | GB 2 | GB 3 | GB 4 | GB 1 | GB5 | UGB1 | GB 1 | GB 1 | GB 1 |
| Ladungsspeicherkapazität (mC/ml) | 1819 | 1659 | 1222 | 910 | 2887 | 2453 | 921 | 37 | 2853 | 30 |
| $\pm$ S.D. | 176,65 | 37,43 | 16,97 | 59,40 | 122,20 | 70,24 | 22,48 | 2,14 | 94,52 | 0,90 |
| Impedanz bei 0,01 Hz ($\Omega$) | 92 | 103 | 93 | 150 | 53 | 62 | 167 | 8946 | 53 | 10636 |
| $\pm$ S.D. | 3,36 | 6,18 | 25,51 | 20,39 | 0,91 | 1,37 | 8,46 | 1143,08 | 1,26 | 1112,39 |

EP 4 251 221 B1

# Tabelle 4-1

## Sequestrierung in % (24 h)

| PO (µmol/ml) nach Quellung | | | 40 | 24 | | |
|---|---|---|---|---|---|---|
| P1 (µmol/ml) nach Quellung | | | 84 | 60 | | |
| P2 (mmol/(g/mol)) | | | 4,5 | 4,5 | | |
| P3 (1/A-2)*10^3 | | | 6,8 | 6,8 | | |
| Hydrogelmaterialtyp | | | GB2-UGB2-UGB1 19 | GB2-UGB2-UGB1 09 | | |
| Protein | Klasse | Pp *10-6 [1/Å2] | kein PEDOT | 500 mV | 0 mV | -500 mV |
| SDF-1α | | 1139 | 98,20 | 25,29 | 72,31 | 47,21 |
| | | | | 54,72 | 13,17 | 4,58 |
| MCP-1 | | 1083 | 95,50 | 9,13 | 33,41 | 0,00 |
| | | | | 64,57 | 28,30 | 0,00 |
| FGF-2 | Positive geladen | 942 | 85,00 | 80,90 | 72,67 | 80,77 |
| | | | | 14,21 | 11,19 | 1,59 |
| NGF-β | | 350 | 100,00 | 70,01 | 94,06 | 74,97 |
| | | | | 21,01 | 2,69 | 2,94 |
| IL-4 | | 315 | 91,20 | 63,56 | 72,15 | 60,43 |
| | | | | 29,73 | 13,58 | 2,41 |
| IL-8 | | 247 | 96,70 | 48,09 | 70,78 | 35,35 |
| | | | | 19,02 | 13,17 | 14,20 |
| PDGF-BB | | 175 | 100,00 | | 86,59 | |
| | | | | | 12,13 | |
| IL-10 | | 27 | 88,40 | 86,46 | 51,59 | 52,69 |
| | | | | 10,97 | 21,16 | 15,85 |
| TNF-α | Neutral | -44 | 87,60 | 65,23 | 46,31 | 15,13 |
| | | | | 30,09 | 20,97 | 4,99 |
| IFN-γ | | -83 | 90,10 | | 66,36 | |
| | | | | | 19,43 | |
| VEGF | | -128 | 79,40 | 0,00 | 64,75 | 0,00 |
| | | | | 0,00 | 18,23 | 0,00 |
| GM-CSF | | -394 | 43,30 | 59,24 | 49,93 | 17,88 |
| | | | | 31,29 | 19,10 | 4,67 |
| MIP-1β | Negativ geladen | -427 | 88,30 | 45,46 | 62,78 | 0,00 |
| | | | | 37,34 | 16,07 | 0,00 |
| EGF | | -814 | 33,50 | 66,99 | 37,15 | 0,00 |
| | | | | 22,56 | 25,41 | 0,00 |

**Tabellen 4-1 bis 4-4:** Bindung bioaktiver Substanzen durch elektrisch leitfähige Hydrogelmaterialien

# Tabelle 4-2

## Sequestrierung in % (24 h)

| P0 (µmol/ml) nach Quellung | | | 1 | | 1 | |
|---|---|---|---|---|---|---|
| P1 (µmol/ml) nach Quellung | | | 2 | | 2 | |
| P2 (mmol/(g/mol)) | | | 4,5 | | 4,5 | |
| P3 (1/A-2)*10^3 | | | 6,8 | | 6,8 | |
| Hydrogelmaterialtyp | | | GB2-UGB2-UGB1 20 | GB2-UGB2-UGB1 10 | | |
| Protein | Klasse | Pp *10-6 [1/Å2] | kein PEDOT | 500 mV | 0 mV | -500 mV |
| SDF-1α | | 1139 | 60,50 | 31,95 | 76,88 | 19,68 |
| | | | | 18,08 | 6,64 | 24,71 |
| MCP-1 | | 1083 | 78,20 | 49,61 | 59,52 | 27,30 |
| | | | | 9,46 | 13,50 | 26,96 |
| FGF-2 | Positive geladen | 942 | 41,60 | 51,27 | 86,10 | 70,33 |
| | | | | 14,44 | 5,89 | 2,79 |
| NGF-β | | 350 | 79,00 | 7,93 | 60,89 | 25,77 |
| | | | | 20,01 | 17,74 | 30,45 |
| IL-4 | | 315 | 45,80 | 61,88 | 69,12 | 48,65 |
| | | | | 12,48 | 16,04 | 24,17 |
| IL-8 | | 247 | 67,80 | 17,06 | 39,94 | 3,69 |
| | | | | 10,10 | 19,34 | 25,96 |
| PDGF-BB | | 175 | 70,10 | 0,00 | 66,38 | 0,00 |
| | | | | 0,00 | 14,25 | 0,00 |
| IL-10 | | 27 | 46,10 | 0,00 | 61,17 | 0,00 |
| | | | | 0,00 | 11,02 | 0,00 |
| TNF-α | Neutral | -44 | 25,10 | 0,00 | 45,94 | 0,00 |
| | | | | 0,00 | 22,62 | 0,00 |
| IFN-γ | | -83 | 64,40 | | 48,30 | |
| | | | | | 18,56 | |
| VEGF | | -128 | 54,00 | 0,00 | 42,42 | 0,00 |
| | | | | 0,00 | 19,03 | 0,00 |
| GM-CSF | | -394 | 0,00 | 78,40 | 77,79 | 44,91 |
| | | | | 5,47 | 6,72 | 25,37 |
| MIP-1β | Negativ geladen | -427 | 63,90 | 26,49 | 78,08 | 28,26 |
| | | | | 16,78 | 5,85 | 25,05 |
| EGF | | -814 | 21,00 | 64,83 | 69,23 | 42,78 |
| | | | | 10,66 | 18,64 | 23,67 |

# Tabelle 4-3

## Sequestrierung in % (24 h)

| | | | | | | |
|---|---|---|---|---|---|---|
| P0 (µmol/ml) nach Quellung | | | 45 | 25 | | |
| P1 (µmol/ml) nach Quellung | | | 81 | 43 | | |
| P2 (mmol/(g/mol)) | | | 3,2 | 3,2 | | |
| P3 (1/A-2)*10^3 | | | -2,5 | -2,5 | | |
| Hydrogelmaterialtyp | | | GB3-UGB2-UGB1 21 | GB3-UGB2-UGB1 11 | | |
| Protein | Klasse | Pp *10-6 [1/Å2] | kein PEDOT | 500 mV | 0 mV | -500 mV |
| SDF-1α | | 1139 | 90,20 | 36,82 | 70,85 | 43,66 |
| | | | | 18,93 | 11,96 | 1,29 |
| MCP-1 | | 1083 | 85,10 | 81,86 | 80,59 | 41,09 |
| | | | | 2,09 | 5,47 | 8,64 |
| FGF-2 | Positive geladen | 942 | 58,10 | 72,49 | 87,01 | 75,62 |
| | | | | 4,43 | 5,01 | 1,82 |
| NGF-β | | 350 | 84,80 | 31,85 | 84,57 | 36,58 |
| | | | | 5,65 | 1,55 | 9,16 |
| IL-4 | | 315 | 78,10 | 76,76 | 78,10 | 68,41 |
| | | | | 0,26 | 6,98 | 4,13 |
| IL-8 | | 247 | 87,20 | 64,68 | 83,85 | 37,80 |
| | | | | 8,60 | 5,78 | 3,25 |
| PDGF-BB | | 175 | 72,60 | 20,63 | 86,18 | 12,97 |
| | | | | 21,61 | 9,59 | 18,34 |
| IL-10 | Neutral | 27 | 33,00 | 58,02 | 72,43 | 4,11 |
| | | | | 7,43 | 7,49 | 16,39 |
| TNF-α | | -44 | 44,80 | 66,33 | 70,98 | 16,58 |
| | | | | 3,30 | 5,90 | 14,23 |
| IFN-γ | | -83 | 76,50 | | | |
| | | | | | | |
| VEGF | | -128 | 41,10 | 0,00 | 67,46 | 0,00 |
| | | | | 0,00 | 33,81 | 0,00 |
| GM-CSF | Negativ geladen | -394 | 14,60 | 74,42 | 64,04 | 60,48 |
| | | | | 11,59 | 7,60 | 5,68 |
| MIP-1β | | -427 | 62,00 | 53,68 | 75,09 | 0,00 |
| | | | | 3,30 | 6,13 | 0,00 |
| EGF | | -814 | 6,00 | 72,68 | 40,45 | 0,00 |
| | | | | 1,82 | 26,51 | 0,00 |

# Tabelle 4-4

## Sequestrierung in % (24 h)

| P0 (µmol/ml) nach Quellung | | | | 33 | | 26 |
|---|---|---|---|---|---|---|
| P1 (µmol/ml) nach Quellung | | | | 12 | | 9 |
| P2 (mmol/(g/mol)) | | | | 0,9 | | 0,9 |
| P3 (1/A-2)*10^3 | | | | 0,7 | | 0,7 |
| Hydrogelmaterialtyp | | | | GB4-UGB2-UGB1 22 | GB4-UGB2-UGB1 12 | |

| Protein | Klasse | Pp *10-6 [1/Å2] | kein PEDOT | 500 mV | 0 mV | -500 mV |
|---|---|---|---|---|---|---|
| SDF-1α | Positive geladen | 1139 | 58,90 | 0,00 | 93,28 | 15,56 |
| | | | | 0,00 | 4,25 | 5,10 |
| MCP-1 | | 1083 | 78,70 | 75,65 | 76,42 | 67,74 |
| | | | | 13,21 | 6,75 | 11,66 |
| FGF-2 | | 942 | 41,00 | 67,27 | 91,78 | 68,40 |
| | | | | 13,83 | 2,52 | 3,48 |
| NGF-β | | 350 | 71,30 | 0,00 | 95,89 | 0,00 |
| | | | | 0,00 | 2,32 | 0,00 |
| IL-4 | | 315 | 48,30 | 55,55 | 78,40 | 73,33 |
| | | | | 5,15 | 9,47 | 5,73 |
| IL-8 | | 247 | 73,00 | 46,30 | 75,79 | 58,38 |
| | | | | 33,42 | 11,06 | 17,55 |
| PDGF-BB | | 175 | 59,00 | 0,00 | 96,86 | 20,54 |
| | | | | 0,00 | 2,18 | 4,45 |
| IL-10 | Neutral | 27 | 17,10 | 32,94 | 71,16 | 29,25 |
| | | | | 21,02 | 5,40 | 17,09 |
| TNF-α | | -44 | 46,50 | 49,24 | 73,96 | 38,62 |
| | | | | 12,25 | 5,88 | 16,43 |
| IFN-γ | | -83 | 70,00 | | | |
| | | | | | | |
| VEGF | | -128 | 56,90 | 0,00 | 76,52 | 0,00 |
| | | | | 0,00 | 13,13 | 0,00 |
| GM-CSF | Negativ geladen | -394 | 9,50 | 63,21 | 64,24 | 74,97 |
| | | | | 3,76 | 5,24 | 11,51 |
| MIP-1β | | -427 | 53,00 | 28,60 | 68,33 | 14,49 |
| | | | | 21,72 | 10,07 | 18,71 |
| EGF | | -814 | 8,50 | 55,89 | 17,59 | 0,00 |
| | | | | 22,89 | 11,65 | 0,00 |

# Tabelle 5-1

## Freisetzung in % (8h)

| P0 (μmol/ml) nach Quellung | | | 24 | | |
|---|---|---|---|---|---|
| P1 (μmol/ml) nach Quellung | | | 60 | | |
| P2 (mmol/(g/mol)) | | | 4,5 | | |
| P3 (1/A-2)*10^3 | | | 6,8 | | |
| Hydrogelmaterialtyp | | | GB2-UGB2-UGB1 09 | | |
| Protein | Klasse | Pp *10-6 [1/Å2] | 500 mV | 0 mV | -500 mV |
| SDF-1α | Positive geladen | 1139 | 0,05 | 0,00 | 0,00 |
| | | | 0,07 | 0,00 | 0,00 |
| MCP-1 | | 1083 | 0,18 | 0,76 | 2,70 |
| | | | 0,09 | 0,24 | 0,05 |
| FGF-2 | | 942 | 0,00 | 0,00 | 0,00 |
| | | | 0,00 | 0,00 | 0,00 |
| NGF-β | | 350 | 0,00 | 0,00 | 0,00 |
| | | | 0,00 | 0,00 | 0,00 |
| IL-4 | | 315 | 0,22 | 0,33 | 0,36 |
| | | | 0,06 | 0,23 | 0,06 |
| IL-8 | | 247 | 0,21 | 0,48 | 3,25 |
| | | | 0,08 | 0,30 | 0,15 |
| PDGF-BB | | 175 | 0,00 | 0,00 | 0,00 |
| | | | 0,00 | 0,00 | 0,00 |
| IL-10 | Neutral | 27 | 0,00 | 0,09 | 0,00 |
| | | | 0,00 | 0,05 | 0,00 |
| TNF-α | | -44 | 0,00 | 1,39 | 5,26 |
| | | | 0,00 | 1,35 | 3,01 |
| IFN-γ | | -83 | 0,00 | 0,00 | 0,77 |
| | | | 0,00 | 0,00 | 1,09 |
| VEGF | | -128 | 0,08 | 0,14 | 1,56 |
| | | | 0,02 | 0,07 | 1,08 |
| GM-CSF | Negativ geladen | -394 | 0,22 | 2,33 | 10,41 |
| | | | 0,03 | 2,41 | 0,47 |
| MIP-1β | | -427 | 0,17 | 0,87 | 8,77 |
| | | | 0,24 | 0,57 | 1,13 |
| EGF | | -814 | 0,86 | 9,12 | 67,18 |
| | | | 0,47 | 3,94 | 49,77 |

**Tabellen 5-1 bis 5-4:** Freisetzung bioaktiver Substanzen durch elektrisch leitfähige Hydrogelmaterialeien bei verschiedenen elektrischen Potentialen

# Tabelle 5-2

## Freisetzung in % (8h)

| PO (µmol/ml) nach Quellung | | | 1 | | |
|---|---|---|---|---|---|
| P1 (µmol/ml) nach Quellung | | | 2 | | |
| P2 (mmol/(g/mol)) | | | 4,5 | | |
| P3 (1/A-2)*10^3 | | | 6,8 | | |
| Hydrogelmaterialtyp | | | GB2-UGB2-UGB1 10 | | |
| Protein | Klasse | Pp *10-6 [1/Å2] | 500 mV | 0 mV | -500 mV |
| SDF-1α | | 1139 | 0,23 | 0,75 | 0,00 |
| | | | 0,33 | 0,73 | 0,00 |
| MCP-1 | | 1083 | 0,32 | 4,35 | 4,58 |
| | | | 0,29 | 2,29 | 1,26 |
| FGF-2 | Positive geladen | 942 | 0,69 | 0,31 | 2,55 |
| | | | 0,67 | 0,20 | 0,47 |
| NGF-β | | 350 | 0,55 | 0,54 | 5,26 |
| | | | 0,78 | 0,15 | 3,20 |
| IL-4 | | 315 | 0,17 | 0,60 | 1,71 |
| | | | 0,14 | 0,24 | 0,16 |
| IL-8 | | 247 | 1,18 | 11,69 | 26,33 |
| | | | 0,87 | 5,97 | 12,08 |
| PDGF-BB | | 175 | 1,55 | 0,98 | 0,00 |
| | | | 2,19 | 1,69 | 0,00 |
| IL-10 | | 27 | 0,02 | 0,03 | 0,14 |
| | | | 0,02 | 0,00 | 0,12 |
| TNF-α | Neutral | -44 | 0,88 | 1,70 | 53,20 |
| | | | 0,85 | 1,05 | 19,68 |
| IFN-γ | | -83 | 10,50 | 17,50 | 57,33 |
| | | | 5,29 | 7,86 | 19,10 |
| VEGF | | -128 | 2,05 | 1,29 | 8,56 |
| | | | 2,64 | 0,80 | 2,68 |
| GM-CSF | | -394 | 0,11 | 0,38 | 5,90 |
| | | | 0,04 | 0,08 | 0,02 |
| MIP-1β | Negativ geladen | -427 | 0,56 | 0,64 | 2,96 |
| | | | 0,17 | 0,33 | 0,58 |
| EGF | | -814 | 0,08 | 1,96 | 72,62 |
| | | | 0,05 | 0,52 | 49,49 |

# Tabelle 5-3

## Freisetzung in % (8h)

| PO (µmol/ml) nach Quellung | 25 |
|---|---|
| P1 (µmol/ml) nach Quellung | 43 |
| P2 (mmol/(g/mol)) | 3,2 |
| P3 (1/A-2)*10^3 | -2,5 |

| Protein | Klasse | Pp *10-6 [1/Å2] | 500 mV | 0 mV | -500 mV |
|---|---|---|---|---|---|
| Hydrogelmaterialtyp | | | GB3-UGB2-UGB1 11 | | |
| SDF-1α | Positive geladen | 1139 | 0,81 | 1,63 | 2,42 |
|  | | | 0,16 | 1,52 | 1,46 |
| MCP-1 | | 1083 | 0,22 | 1,33 | 2,10 |
|  | | | 0,01 | 0,25 | 1,33 |
| FGF-2 | | 942 | 0,00 | 0,01 | 0,08 |
|  | | | 0,00 | 0,03 | 0,11 |
| NGF-β | | 350 | 0,00 | 0,21 | 0,00 |
|  | | | 0,00 | 0,36 | 0,00 |
| IL-4 | | 315 | 1,34 | 2,23 | 1,66 |
|  | | | 0,58 | 1,41 | 0,59 |
| IL-8 | | 247 | 0,30 | 1,43 | 1,88 |
|  | | | 0,02 | 0,28 | 0,84 |
| PDGF-BB | | 175 | 0,00 | 0,00 | 0,00 |
|  | | | 0,00 | 0,00 | 0,00 |
| IL-10 | Neutral | 27 | 0,08 | 0,30 | 0,43 |
|  | | | 0,02 | 0,03 | 0,27 |
| TNF-α | | -44 | 0,07 | 0,83 | 2,85 |
|  | | | 0,07 | 0,16 | 2,82 |
| IFN-γ | | -83 | | | |
|  | | | | | |
| VEGF | | -128 | 1,70 | 2,96 | 13,74 |
|  | | | 0,77 | 2,29 | 11,01 |
| GM-CSF | Negativ geladen | -394 | 0,50 | 4,35 | 12,29 |
|  | | | 0,20 | 0,24 | 9,19 |
| MIP-1β | | -427 | 0,93 | 2,92 | 3,47 |
|  | | | 0,25 | 0,18 | 1,39 |
| EGF | | -814 | 1,19 | 94,10 | 100,00 |
|  | | | 0,40 | 139,88 | 128,28 |

# Tabelle 5-4

**Freisetzung in % (8h)**

| P0 (µmol/ml) nach Quellung | | | 26 | | |
|---|---|---|---|---|---|
| P1 (µmol/ml) nach Quellung | | | 9 | | |
| P2 (mmol/(g/mol)) | | | 0,9 | | |
| P3 (1/A-2)*10^3 | | | 0,7 | | |
| Hydrogelmaterialtyp | | | GB4-UGB2-UGB1 12 | | |
| Protein | Klasse | Pp *10-6 [1/Å2] | 500 mV | 0 mV | -500 mV |
| SDF-1α | Positive geladen | 1139 | 0,06 | 0,23 | 0,21 |
| | | | 0,08 | 0,17 | 0,01 |
| MCP-1 | | 1083 | 0,16 | 1,35 | 2,08 |
| | | | 0,01 | 0,40 | 1,02 |
| FGF-2 | | 942 | 0,00 | 0,02 | 0,13 |
| | | | 0,00 | 0,02 | 0,07 |
| NGF-β | | 350 | 0,00 | 0,09 | 0,02 |
| | | | 0,00 | 0,08 | 0,03 |
| IL-4 | | 315 | 0,29 | 0,83 | 1,05 |
| | | | 0,05 | 0,20 | 0,54 |
| IL-8 | | 247 | 0,16 | 1,18 | 3,68 |
| | | | 0,09 | 0,70 | 1,68 |
| PDGF-BB | | 175 | 0,09 | 0,08 | 0,05 |
| | | | 0,12 | 0,07 | 0,07 |
| IL-10 | Neutral | 27 | 0,04 | 0,26 | 0,37 |
| | | | 0,01 | 0,05 | 0,16 |
| TNF-α | | -44 | 0,20 | 0,75 | 1,38 |
| | | | 0,12 | 0,34 | 0,65 |
| IFN-γ | | -83 | | | |
| | | | | | |
| VEGF | | -128 | 0,08 | 0,60 | 0,94 |
| | | | 0,03 | 0,42 | 0,63 |
| GM-CSF | Negativ geladen | -394 | 0,22 | 3,65 | 5,76 |
| | | | 0,17 | 0,28 | 2,93 |
| MIP-1β | | -427 | 0,10 | 2,17 | 6,34 |
| | | | 0,14 | 0,67 | 4,18 |
| EGF | | -814 | 3,44 | 73,54 | 100,00 |
| | | | 3,45 | 22,11 | |

# Tabelle 6

## PC12 Kultur unter Verwendung von Cryogelen GB1-UGB2 17

| Zellumfang (µm/Zelle) | | | | | |
|---|---|---|---|---|---|
| t (d) | | 5 | | 7 | |
| | | Mittelwert | S.D. | Mittelwert | S.D. |
| NGF-β | ohne | 7,92 | 1,32 | 4,32 | 0,46 |
| | im Medium | 18,49 | 1,53 | 19,58 | 4,34 |
| | im Gel | 10,25 | 0,51 | 9,55 | 1,67 |
| | im Gel, reduziertes Medium | 13,1 | 2,14 | 17,77 | 4,45 |
| Zellzahl (Zellen/cm²) | | | | | |
| t (d) | | 5 | | 7 | |
| | | Mittelwert | S.D. | Mittelwert | S.D. |
| NGF-β | ohne | 919,17 | 304,79 | 4553,24 | 727,33 |
| | im Medium | 1590,24 | 336,16 | 1934,93 | 686,48 |
| | im Gel | 2138,08 | 359,01 | 2852,43 | 1222,45 |
| | im Gel, reduziertes Medium | 1501,98 | 365,23 | 1523,63 | 265,07 |

**Tabelle 6:** Biokompatibilität von elektrisch leitfähigen Hydrogelmaterialien gegenüber PC12 Zellen und daraus differenzierter Neuron-artigen Zellen

| Elektrische Eigenschaften | elektr. Charakt. | elektr. Charakt. | elektr. Charakt. | elektr. Charakt. | elektr. Charakt. | elektr. Charakt. | elektr. Charakt. | elektr. Charakt. | Multiplex | Multiplex | Multiplex | Multiplex |
|---|---|---|---|---|---|---|---|---|---|---|---|---|
| Netzwerk Typ | 1 | 2 | 3 | 4 | 5 | 6 | 7 | 8 | 9 | 10 | 11 | 12 |
| P0 (µmol/ml) after swelling | 46,00 | 4,00 | 2,00 | 1,00 | 51,00 | 5,00 | 2,00 | 1,00 | 24,00 | 1,00 | 25,00 | 26,00 |
| P1 (µmol/ml) before swelling | 142,00 | 17,00 | 5,70 | 1,70 | 143 | 18 | 6,10 | 1,80 | 91 | 3,00 | 61 | 12 |
| P1 (µmol/ml) after swelling | 110,00 | 11,00 | 4,00 | 2,00 | 128,00 | 12,00 | 5,00 | 2,00 | 60,00 | 2,00 | 43,00 | 9,00 |
| P2 (mmol/(g/mol)) | 4,5 | 4,5 | 4,5 | 4,5 | 4,5 | 4,5 | 4,5 | 4,5 | 4,5 | 4,5 | 3,2 | 0,9 |
| P3 *10^-3 (1/A^2) | -5,9 | -5,9 | -5,9 | -5,9 | 6,8 | 6,8 | 6,8 | 6,8 | 6,8 | 6,8 | -2,5 | 0,7 |
| Fractional charge | 0,32 | 0,32 | 0,32 | 0,32 | 0,26 | 0,26 | 0,26 | 0,26 | 0,26 | 0,26 | 0,30 | 0,30 |
| SC % | 5,00% | 3.3% (3.41%) | 3.3% (3.34%) | 3.3% (3.31%) | 2,60% | 3.3% (3.5%) | 3.3% (3.37%) | 3.3% (3.32%) | 3.3% (4.3%) | 3.3% (3.33%) | 3.3% (4.41%) | 3.3% (3.87%) |
| % Polymer Mal | 100,0% | 15,0% | 5,0% | 1,5% | 100% | 12% | 4% | 1,2% | 60% | 2% | 45% | 45% |
| Swelling degree | 1,29 | 1,58 | 1,46 | 1,11 | 1,11 | 1,47 | 1,11 | 1,11 | 1,50 | 1,30 | 1,43 | 1,39 |
| geladener Polymerbausteine | HEP | HEP | HEP | HEP | PSSA 3:1 | PSSA 3:1 | PSSA 3:1 | PSSA 3:1 | PSSA 3:1 | PSSA 3:1 | AEHS50% | AEHS10% |
| geladener Polymerbausteine | GB 1 | GB 1 | GB 1 | GB 1 | GB 2 | GB 2 | GB 2 | GB 2 | GB 2 | GB 2 | GB 3 | GB 4 |
| CSC (mC/ml | 3040 | 2350 | 1811 | 1505 | 2730 | 3040 | 2140 | 1894 | 1819 | 1659 | 1222 | 910 |
| ± S.D. | 28,28 | 14,14 | 21,21 | 128,69 | 14,14 | 56,57 | 28,28 | 82,02 | 176,65 | 37,43 | 16,97 | 59,40 |
| Impedanz bei 0,01 Hz (Ω) | 30 | 42 | 81 | 93 | 84 | 63 | 80 | 94 | 92 | 103 | 93 | 150 |
| ± S.D. | 1,52 | 2,12 | 4,06 | 4,65 | 2,95 | 2,41 | 5,52 | 4,55 | 3,36 | 6,18 | 25,51 | 20,39 |

| NGF Aufnahme und Freisetzung | NGF Aufnahme und Freisetzung | Kontrolle elektr. Charakt. | | | Cryogele | |
|---|---|---|---|---|---|---|
| 13 | 14 | 15 | 16 | | 17 | 18 |
| 33,60 | 5,58 | 0,00 | 33,60 | | | |
| 101 | 17 | 0 | 101 | | 101 | 101 |
| 81,00 | 9,00 | 0,00 | 81,00 | | | |
| 4,51 | 1,78 | 0 | 4,51 | | 4,51 | 4,51 |
| -5,9 | -6,75 | -2,82 | -5,9 | | -5,9 | -5,9 |
| 0,32 | 0,32 | 0,00 | 0,32 | | 0,32 | 0,32 |
| 3,30% | 3.3% (3.41%) | 3,30% | 3,30% | | 3,30% | 3,30% |
| | 15% | 0% | | | | |
| 1,25 | 1,58 | 1,25 | 1,25 | | | |
| HEP | 6-O,N DHEP | PEG-PEG | HEP | | HEP | HEP |
| GB 1 | GB5 | UGB1 | GB 1 | | GB 1 | GB 1 |
| 2887 | 2453 | 921 | 37 | | 2853 | 30 |
| 122,20 | 70,24 | 22,48 | 2,14 | | 94,52 | 0,90 |
| 53 | 62 | 167 | 8946 | | 53 | 10636 |
| 0,91 | 1,37 | 8,46 | 1143,08 | | 1,26 | 1112,39 |

## Elektrische Eigenschaften 1

| Swelling degree | 1,293333 | 1,578629 | 1,455293 | 1,11 | 1,113333 | 1,472055 | 1,111092 | 1,111092 | 1,5049835 | 1,3 | 1,43 | 1,39 | 1,25 |
|---|---|---|---|---|---|---|---|---|---|---|---|---|---|
| P0 (µmol/ml) after swelling | 45,695888 | 4,370881 | 1,622354 | 0,664865 | 51,46708 | 4,89112 | 2,178937 | 0,653411 | 24,12784 | 0,93 | 25,393007 | 26,123741 | 0 |
| P1 (µmol/ml) after swelling | 109,79 | 10,77 | 3,92 | 1,53 | 128,44 | 12,23 | 5,49 | 1,62 | 60,47 | 2,31 | 42,66 | 8,63 | 0 |
| P2 (mmol/(g/mol)) | 4,5 | 4,5 | 4,5 | 4,5 | 4,5 | 4,5 | 4,5 | 4,5 | 4,5 | 4,5 | 3,23 | 0,94 | 0 |
| P3 *10^-3 (1/A^2) | -5,9 | -5,9 | -5,9 | -5,9 | 6,8 | 6,8 | 6,8 | 6,8 | 6,8 | 6,8 | -2,5 | 0,7 | -2,82 |
| Charged polymer | HEP | HEP | HEP | HEP | PSSA 3:1 | PSSA 3:1 | PSSA 3:1 | PSSA 3:1 | PSSA 3:1 | PSSA 3:1 | AEHS50% | AEHS10% | PEG-PEG |
| CSC (mC/ml) | 3040 | 2350 | 1811 | 1505 | 2730 | 3040 | 2140 | 1894 | 1818,6667 | 1659,3333 | 1222 | 910 | 920,6666667 |
| ± S.D. | 28,28427 | 14,14214 | 21,2132 | 128,6934 | 14,14214 | 56,56854 | 28,28427 | 82,02439 | 176,65031 | 37,434387 | 16,970563 | 59,39697 | 22,4796204 |
| Impedanz bei 0,01 Hz (Ω) | 30,322 | 42,341 | 81,192 | 93,012 | 41,508 | 36,341 | 67,777 | 74,03 | 91,916667 | 102,871 | 93,293 | 149,529 | 166,8166667 |
| ± S.D. | 1,5161 | 2,11705 | 4,0596 | 4,6506 | 2,0754 | 1,81705 | 3,38885 | 3,7015 | 3,3589459 | 6,1818815 | 25,510998 | 20,391545 | 8,461349203 |

# Elektrische Eigenschaften 1

| | Bulk | Cryo | undoped control | Bulk | Cryo | P1 low | |
|---|---|---|---|---|---|---|---|
| Swelling degree | 1,25 | 1,6 | 1,25 | 1,25 | 1,6 | 1,578629221 | 1,25 |
| P1 (µmol/ml) after swelling | 81 | 81 | 0 | 81 | 81 | 10,77 | 9 |
| P2 (mmol/(g/mol)) | 4,5 | 4,5 | 0 | 4,5 | 4,5 | 4,5 | 1,78 |
| P3 *10^-3 (1/A^2) | -5,9 | -5,9 | -2,82 | -5,9 | -5,9 | -5,9 | -6,75 |
| Charged polymer | HEP | HEP | PEG-PEG | HEP | HEP | HEP | 6-O,N DHEP |
| CSC (mC/ml) | 36,66666667 | 30,13333333 | 920,6666667 | 2886,666667 | 2853,333333 | 2350 | 2453,333333 |
| ± S.D. | 2,138535324 | 0,901849951 | 22,4796204 | 122,2020185 | 94,51631253 | 14,14214 | 70,23769169 |
| Impedanz at 0,01 Hz (Ω) | 8946 | 10635,66667 | 166,8166667 | 53,381 | 52,62433333 | 42,341 | 61,76 |
| ± S.D. | 1143,083986 | 1112,387672 | 8,461349203 | 0,912167748 | 1,263355189 | 2,11705 | 1,372269653 |
| | w/o PEDOT | w/o PEDOT | | | | | |

### Sequestrierung in % (24 h)

| | 19 | 9 | 20 | 10 | 21 | 11 | 22 | 12 |
|---|---|---|---|---|---|---|---|---|
| Swelling degree | | 1,50 | | 1,30 | | 1,43 | | 1,39 |
| P0 (µmol/ml) after swelling | | 24 | | 1 | | 25 | | 26 |
| P1 (µmol/ml) after swelling | 70 | 60 | 2 | 2 | 70 | 43 | 15 | 9 |
| P2 (mmol/(g/mol)) | 4,5 | 4,5 | 4,5 | 4,5 | 3,2 | 3,2 | 0,9 | 0,9 |
| P3 (1/A-2)*10^3 | 6,8 | 6,8 | 6,8 | 6,8 | -2,5 | -2,5 | 0,7 | 0,7 |
| geladener Polymerbaustein | PSSA3:1 | PSSA3:1 | PSSA3:1 | PSSA3:1 | AEHS50 | AEHS50% | AEHS10 | AEHS10% |

| IEP | Protein | Klasse | Charge | no PEDOT | 500 mV | 0 mV | -500 mV | no PEDOT | 500 mV | 0 mV | -500 mV | no PEDOT | 500 mV | 0 mV | -500 mV | no PEDOT | 500 mV | 0 mV | -500 mV |
|---|---|---|---|---|---|---|---|---|---|---|---|---|---|---|---|---|---|---|---|
| 10,1 | SDF-1α | | 1139 | 98,2 | 25,3 | 72,3 | 47,2 | 60,5 | 32,0 | 76,9 | 19,7 | 90,2 | 36,8 | 70,9 | 43,7 | 58,9 | 0,0 | 93,3 | 15,6 |
| 9,4 | MCP-1 | | 1083 | 95,5 | 9,1 | 33,4 | 0,0 | 78,2 | 49,6 | 59,5 | 27,3 | 85,1 | 81,9 | 80,6 | 41,1 | 78,7 | 75,7 | 76,4 | 67,7 |
| 9,58 | FGF-2 | | 942 | 85,0 | 80,9 | 72,7 | 80,8 | 41,6 | 51,3 | 86,1 | 70,3 | 58,1 | 72,5 | 87,0 | 75,6 | 41,0 | 67,3 | 91,8 | 68,4 |
| 9 | NGF-β | | 350 | 100,0 | 70,0 | 94,1 | 75,0 | 79,0 | 7,9 | 60,9 | 25,8 | 84,8 | 31,9 | 84,6 | 36,6 | 71,3 | 0,0 | 95,9 | 0,0 |
| 9,3 | IL-4 | | 315 | 91,2 | 63,6 | 72,2 | 60,4 | 45,8 | 61,9 | 69,1 | 48,6 | 78,1 | 76,8 | 78,1 | 68,4 | 48,3 | 55,5 | 78,4 | 73,3 |
| 9,2 | IL-8 | | 247 | 96,7 | 48,1 | 70,8 | 35,4 | 67,8 | 17,1 | 39,9 | 3,7 | 87,2 | 64,7 | 83,8 | 37,8 | 73,0 | 46,3 | 75,8 | 58,4 |
| 9,38 | PDGF-BB | Positive gel | 175 | 100,0 | | 86,6 | | 70,1 | 0,0 | 66,4 | 0,0 | 72,6 | 20,6 | 86,2 | 13,0 | 59,0 | 0,0 | 96,9 | 20,5 |
| 7,6 | IL-10 | | 27 | 88,4 | 86,5 | 51,6 | 52,7 | 46,1 | 0,0 | 61,2 | 0,0 | 33,0 | 58,0 | 72,4 | 4,1 | 17,1 | 32,9 | 71,2 | 29,3 |
| 7 | TNF-α | | -44 | 87,6 | 65,2 | 46,3 | 15,1 | 25,1 | 0,0 | 45,9 | 0,0 | 44,8 | 66,3 | 71,0 | 16,6 | 46,5 | 49,2 | 74,0 | 38,6 |
| 9,5 | IFN-γ | | -83 | 90,1 | | 66,4 | | 64,4 | | 48,3 | | 76,5 | | | | 70,0 | | | |
| 7,6 | VEGF | Neutral | -128 | 79,4 | 0,0 | 64,7 | 0,0 | 54,0 | 0,0 | 42,4 | 0,0 | 41,1 | 0,0 | 67,5 | 0,0 | 56,9 | 0,0 | 76,5 | 0,0 |
| 5,2 | GM-CSF | | -394 | 43,3 | 59,2 | 49,9 | 17,9 | 0,0 | 78,4 | 77,8 | 44,9 | 14,6 | 74,4 | 64,0 | 60,5 | 9,5 | 63,2 | 64,2 | 75,0 |
| 4,8 | MIP-1β | | -427 | 88,3 | 45,5 | 62,8 | 0,0 | 63,9 | 26,5 | 78,1 | 28,3 | 62,0 | 53,7 | 75,1 | 0,0 | 53,0 | 28,6 | 68,3 | 14,5 |
| 4,8 | EGF | Negativ gel | -814 | 33,5 | 67,0 | 37,1 | 0,0 | 21,0 | 64,8 | 69,2 | 42,8 | 6,0 | 72,7 | 40,4 | 0,0 | 8,5 | 55,9 | 17,6 | 0,0 |

**Sequestrierung in % ±S.D. (24 h)**

| IEP | Protein | Klasse | Charge | | | | | | | | | | | | | | | | |
|---|---|---|---|---|---|---|---|---|---|---|---|---|---|---|---|---|---|---|---|
| 10,1 | SDF-1α | Positive geladen | 1139 | 98,20 | 25,29 | 72,31 | 47,21 | 60,50 | 31,95 | 76,88 | 19,68 | 90,20 | 36,82 | 70,85 | 43,66 | 58,90 | 0,00 | 93,28 | 15,56 |
| | | | | | 54,72 | 13,17 | 4,58 | | 18,08 | 6,64 | 24,71 | | 18,93 | 11,96 | 1,29 | | 0,00 | 4,25 | 5,10 |
| 9,4 | MCP-1 | | 1083 | 95,50 | 9,13 | 33,41 | 0,00 | 78,20 | 49,61 | 59,52 | 27,30 | 85,10 | 81,86 | 80,59 | 41,09 | 78,70 | 75,65 | 76,42 | 67,74 |
| | | | | | 64,57 | 28,30 | 0,00 | | 9,46 | 13,50 | 26,96 | | 2,09 | 5,47 | 8,64 | | 13,21 | 6,75 | 11,66 |
| 9,58 | FGF-2 | | 942 | 85,00 | 80,90 | 72,67 | 80,77 | 41,60 | 51,27 | 86,10 | 70,33 | 58,10 | 72,49 | 87,01 | 75,62 | 41,00 | 67,27 | 91,78 | 68,40 |
| | | | | | 14,21 | 11,19 | 1,59 | | 14,44 | 5,89 | 2,79 | | 4,43 | 5,01 | 1,82 | | 13,83 | 2,52 | 3,48 |
| 9 | NGF-β | | 350 | 100,00 | 70,01 | 94,06 | 74,97 | 79,00 | 7,93 | 60,89 | 25,77 | 84,80 | 31,85 | 84,57 | 36,58 | 71,30 | 0,00 | 95,89 | 0,00 |
| | | | | | 21,01 | 2,69 | 2,94 | | 20,01 | 17,74 | 30,45 | | 5,65 | 1,55 | 9,16 | | 0,00 | 2,32 | 0,00 |
| 9,3 | IL-4 | | 315 | 91,20 | 63,56 | 69,12 | 60,43 | 45,80 | 61,88 | 69,12 | 48,65 | 78,10 | 76,76 | 78,10 | 68,41 | 48,30 | 55,55 | 78,40 | 73,33 |
| | | | | | 29,73 | 13,58 | 2,41 | | 12,48 | 16,04 | 24,17 | | 0,26 | 6,98 | 4,13 | | 5,15 | 9,47 | 5,73 |
| 9,2 | IL-8 | | 247 | 96,70 | 48,09 | 70,78 | 35,35 | 67,80 | 17,06 | 39,94 | 3,69 | 87,20 | 64,68 | 83,85 | 37,80 | 73,00 | 46,30 | 75,79 | 58,38 |
| | | | | | 19,02 | 13,17 | 14,20 | | 10,10 | 19,34 | 25,96 | | 8,60 | 5,78 | 3,25 | | 33,42 | 11,06 | 17,55 |
| 9,38 | PDGF-BB | | 175 | 100,00 | | 86,59 | | 70,10 | 0,00 | 66,38 | 0,00 | 72,60 | 20,63 | 86,18 | 12,97 | 59,00 | 0,00 | 96,86 | 20,54 |
| | | | | | | 12,13 | | | 0,00 | 14,25 | 0,00 | | 21,61 | 9,59 | 18,34 | | 0,00 | 2,18 | 4,45 |
| 7,6 | IL-10 | Neutral | 27 | 88,40 | 86,46 | 51,59 | 52,69 | 46,10 | 0,00 | 61,17 | 0,00 | 33,00 | 58,02 | 72,43 | 4,11 | 17,10 | 32,94 | 71,16 | 29,25 |
| | | | | | 10,97 | 21,16 | 15,85 | | 0,00 | 11,02 | 0,00 | | 7,43 | 7,49 | 16,39 | | 21,02 | 5,40 | 17,09 |
| 7 | TNF-α | | -44 | 87,60 | 65,23 | 46,31 | 15,13 | 25,10 | 0,00 | 45,94 | 0,00 | 44,80 | 66,33 | 70,98 | 16,58 | 46,50 | 49,24 | 73,96 | 38,62 |
| | | | | | 30,09 | 20,97 | 4,99 | | 0,00 | 22,62 | 0,00 | | 3,30 | 5,90 | 14,23 | | 12,25 | 5,88 | 16,43 |
| 9,5 | IFN-γ | | -83 | 90,10 | | 66,36 | | 64,40 | | 48,30 | | 76,50 | | | | 70,00 | | | |
| | | | | | | 19,43 | | | | 18,56 | | | | | | | | | |
| 7,6 | VEGF | | -128 | 79,40 | 0,00 | 64,75 | 0,00 | 54,00 | 0,00 | 42,42 | 0,00 | 41,10 | 0,00 | 67,46 | 0,00 | 56,90 | 0,00 | 76,52 | 0,00 |
| | | | | | 0,00 | 18,23 | 0,00 | | 0,00 | 19,03 | 0,00 | | 0,00 | 33,81 | 0,00 | | 0,00 | 13,13 | 0,00 |
| 5,2 | GM-CSF | Negativ geladen | -394 | 43,30 | 59,24 | 49,93 | 17,88 | 0,00 | 78,40 | 77,79 | 44,91 | 14,60 | 74,42 | 64,04 | 60,48 | 9,50 | 63,21 | 64,24 | 74,97 |
| | | | | | 31,29 | 19,10 | 4,67 | | 5,47 | 6,72 | 25,37 | | 11,59 | 7,60 | 5,68 | | 3,76 | 5,24 | 11,51 |
| 4,8 | MIP-1β | | -427 | 88,30 | 45,46 | 62,78 | 0,00 | 63,90 | 26,49 | 78,08 | 28,26 | 62,00 | 53,68 | 75,09 | 0,00 | 53,00 | 28,60 | 68,33 | 14,49 |
| | | | | | 37,34 | 16,07 | 0,00 | | 16,78 | 5,85 | 25,05 | | 3,30 | 6,13 | 0,00 | | 21,72 | 10,07 | 18,71 |
| 4,8 | EGF | | -814 | 33,50 | 66,99 | 37,15 | 0,00 | 21,00 | 64,83 | 69,23 | 42,78 | 6,00 | 72,68 | 40,45 | 0,00 | 8,50 | 55,89 | 17,59 | 0,00 |
| | | | | | 22,56 | 25,41 | 0,00 | | 10,66 | 18,64 | 23,67 | | 1,82 | 26,51 | 0,00 | | 22,89 | 11,65 | 0,00 |

## Freisetzung 1

**Freisetzung in % (8h)**

| | | | | 9 | | | 10 | | | 11 | | | 12 | | |
|---|---|---|---|---|---|---|---|---|---|---|---|---|---|---|---|
| Swelling degree | | | | 1,50 | | | 1,30 | | | 1,43 | | | 1,39 | | |
| P0 (µmol/ml) after swelling | | | | 24 | | | 1 | | | 25 | | | 26 | | |
| P1 (µmol/ml) after swelling | | | | 60 | | | 2 | | | 43 | | | 9 | | |
| P2 (mmol/(g/mol)) | | | | 4,50 | | | 4,50 | | | 3,23 | | | 0,94 | | |
| P3 $(1/A{-}2)*10^3$ | | | | 6,8 | | | 6,8 | | | -2,5 | | | 0,7 | | |
| geladener Polymerbaustein | | | | PSSA3:1 | | | PSSA3:1 | | | AEHS50% | | | AEHS10% | | |
| IEP | Protein | Klasse | Charge/SA $(x10^6)$ $(1/A^2)$ | 500 mV | 0 mV | -500 mV | 500 mV | 0 mV | -500 mV | 500 mV | 0 mV | -500 mV | 500 mV | 0 mV | -500 mV |
| 10,1 | SDF-1α | Positiv geladen | 1139 | 0,0 | 0,0 | 0,0 | 0,2 | 0,8 | 0,0 | 0,8 | 1,6 | 2,4 | 0,1 | 0,2 | 0,2 |
| 9,4 | MCP-1 | | 1083 | 0,2 | 0,8 | 2,7 | 0,3 | 4,4 | 4,6 | 0,2 | 1,3 | 2,1 | 0,2 | 1,4 | 2,1 |
| 9,58 | FGF-2 | | 942 | 0,0 | 0,0 | 0,0 | 0,7 | 0,3 | 2,6 | 0,0 | 0,0 | 0,1 | 0,0 | 0,0 | 0,1 |
| 9 | NGF-β | | 350 | 0,0 | 0,0 | 0,0 | 0,6 | 0,5 | 5,3 | 0,0 | 0,2 | 0,0 | 0,0 | 0,1 | 0,0 |
| 9,3 | IL-4 | | 315 | 0,2 | 0,3 | 0,4 | 0,2 | 0,6 | 1,7 | 1,3 | 2,2 | 1,7 | 0,3 | 0,8 | 1,1 |
| 9,2 | IL-8 | | 247 | 0,2 | 0,5 | 3,2 | 1,2 | 11,7 | 26,3 | 0,3 | 1,4 | 1,9 | 0,2 | 1,2 | 3,7 |
| 9,38 | PDGF-BB | | 175 | 0,0 | 0,0 | 0,0 | 1,6 | 1,0 | 0,0 | 0,0 | 0,0 | 0,0 | 0,1 | 0,1 | 0,1 |
| 7,6 | IL-10 | Neutral | 27 | 0,0 | 0,1 | 0,0 | 0,0 | 0,0 | 0,1 | 0,1 | 0,3 | 0,4 | 0,0 | 0,3 | 0,4 |
| 7 | TNF-α | | -44 | 0,0 | 1,4 | 5,3 | 0,9 | 1,7 | 53,2 | 0,1 | 0,8 | 2,9 | 0,2 | 0,8 | 1,4 |
| 9,5 | IFN-γ | | -83 | 0,0 | 0,0 | 0,8 | 10,5 | 17,5 | 57,3 | | | | | | |
| 7,6 | VEGF | | -128 | 0,1 | 0,1 | 1,6 | 2,1 | 1,3 | 8,6 | 1,7 | 3,0 | 13,7 | 0,2 | 0,6 | 0,9 |
| 5,2 | GM-CSF | Negativ geladen | -394 | 0,2 | 2,3 | 10,4 | 0,1 | 0,4 | 5,9 | 0,5 | 4,3 | 12,3 | 0,2 | 3,7 | 5,8 |
| 4,8 | MIP-1β | | -427 | 0,2 | 0,9 | 8,8 | 0,6 | 0,6 | 3,0 | 0,9 | 2,9 | 3,5 | 0,1 | 2,2 | 6,3 |
| 4,8 | EGF | | -814 | 0,9 | 9,1 | 67,2 | 0,1 | 2,0 | 72,6 | 1,2 | 94,1 | 100,0 | 3,4 | 73,5 | 100,0 |

## Freisetzung 1

| Freisetzung in % ±S.D. (8h) | | | | | | | | | | | | | | | |
|---|---|---|---|---|---|---|---|---|---|---|---|---|---|---|---|
| 10,1 | SDF-1α | | 1139 | 0,05 | 0,00 | 0,00 | 0,23 | 0,75 | 0,00 | 0,81 | 1,63 | 2,42 | 0,06 | 0,23 | 0,21 |
| | | | | 0,07 | 0,00 | 0,00 | 0,33 | 0,73 | 0,00 | 0,16 | 1,52 | 1,46 | 0,08 | 0,17 | 0,01 |
| 9,4 | MCP-1 | | 1083 | 0,18 | 0,76 | 2,70 | 0,32 | 4,35 | 4,58 | 0,22 | 1,33 | 2,10 | 0,16 | 1,35 | 2,08 |
| | | | | 0,09 | 0,24 | 0,05 | 0,29 | 2,29 | 1,26 | 0,01 | 0,25 | 1,33 | 0,01 | 0,40 | 1,02 |
| 9,58 | FGF-2 | Positive geladen | 942 | 0,00 | 0,00 | 0,00 | 0,69 | 0,31 | 2,55 | 0,00 | 0,01 | 0,08 | 0,00 | 0,02 | 0,13 |
| | | | | 0,00 | 0,00 | 0,00 | 0,67 | 0,20 | 0,47 | 0,00 | 0,03 | 0,11 | 0,00 | 0,02 | 0,07 |
| 9 | NGF-β | | 350 | 0,00 | 0,00 | 0,00 | 0,55 | 0,54 | 5,26 | 0,00 | 0,21 | 0,00 | 0,00 | 0,09 | 0,02 |
| | | | | 0,00 | 0,00 | 0,00 | 0,78 | 0,15 | 3,20 | 0,00 | 0,36 | 0,00 | 0,00 | 0,08 | 0,03 |
| 9,3 | IL-4 | | 315 | 0,22 | 0,33 | 0,36 | 0,17 | 0,60 | 1,71 | 1,34 | 2,23 | 1,66 | 0,29 | 0,83 | 1,05 |
| | | | | 0,06 | 0,23 | 0,06 | 0,14 | 0,24 | 0,16 | 0,58 | 1,41 | 0,59 | 0,05 | 0,20 | 0,54 |
| 9,2 | IL-8 | | 247 | 0,21 | 0,48 | 3,25 | 1,18 | 11,69 | 26,33 | 0,30 | 1,43 | 1,88 | 0,16 | 1,18 | 3,68 |
| | | | | 0,08 | 0,30 | 0,15 | 0,87 | 5,97 | 12,08 | 0,02 | 0,28 | 0,84 | 0,09 | 0,70 | 1,68 |
| 9,38 | PDGF-BB | | 175 | 0,00 | 0,00 | 0,00 | 1,55 | 0,98 | 0,00 | 0,00 | 0,00 | 0,00 | 0,09 | 0,08 | 0,05 |
| | | | | 0,00 | 0,00 | 0,00 | 2,19 | 1,69 | 0,00 | 0,00 | 0,00 | 0,00 | 0,12 | 0,07 | 0,07 |
| 7,6 | IL-10 | | 27 | 0,00 | 0,09 | 0,00 | 0,02 | 0,03 | 0,14 | 0,08 | 0,30 | 0,43 | 0,04 | 0,26 | 0,37 |
| | | | | 0,00 | 0,05 | 0,00 | 0,02 | 0,00 | 0,12 | 0,02 | 0,03 | 0,27 | 0,01 | 0,05 | 0,16 |
| 7 | TNF-α | Neutral | -44 | 0,00 | 1,39 | 5,26 | 0,88 | 1,70 | 53,20 | 0,07 | 0,83 | 2,85 | 0,20 | 0,75 | 1,38 |
| | | | | 0,00 | 1,35 | 3,01 | 0,85 | 1,05 | 19,68 | 0,07 | 0,16 | 2,82 | 0,12 | 0,34 | 0,65 |
| 9,5 | IFN-γ | | -83 | 0,00 | 0,00 | 0,77 | 10,50 | 17,50 | 57,33 | | | | | | |
| | | | | 0,00 | 0,00 | 1,09 | 5,29 | 7,86 | 19,10 | | | | | | |
| 7,6 | VEGF | | -128 | 0,08 | 0,14 | 1,56 | 2,05 | 1,29 | 8,56 | 1,70 | 2,96 | 13,74 | 0,08 | 0,60 | 0,94 |
| | | | | 0,02 | 0,07 | 1,08 | 2,64 | 0,80 | 2,68 | 0,77 | 2,29 | 11,01 | 0,03 | 0,42 | 0,63 |
| 5,2 | GM-CSF | | -394 | 0,22 | 2,33 | 10,41 | 0,11 | 0,38 | 5,90 | 0,50 | 4,35 | 12,29 | 0,22 | 3,65 | 5,76 |
| | | | | 0,03 | 2,41 | 0,47 | 0,04 | 0,08 | 0,02 | 0,20 | 0,24 | 9,19 | 0,17 | 0,28 | 2,93 |
| 4,8 | MIP-1β | Negativ geladen | -427 | 0,17 | 0,87 | 8,77 | 0,56 | 0,64 | 2,96 | 0,93 | 2,92 | 3,47 | 0,10 | 2,17 | 6,34 |
| | | | | 0,24 | 0,57 | 1,13 | 0,17 | 0,33 | 0,58 | 0,25 | 0,18 | 1,39 | 0,14 | 0,67 | 4,18 |
| 4,8 | EGF | | -814 | 0,86 | 9,12 | 67,18 | 0,08 | 1,96 | 72,62 | 1,19 | 94,10 | 100,00 | 3,44 | 73,54 | 100,00 |
| | | | | 0,47 | 3,94 | 49,77 | 0,05 | 0,52 | 49,49 | 0,40 | 139,88 | 128,28 | 3,45 | 22,11 | out of dete |

## Patentansprüche

1. Verfahren zur Erfassung und Beeinflussung einer Aufnahme von bioaktiven Molekülen (6) in einem Hydrogelmaterial (1) und/oder Freisetzung von bioaktiven Molekülen (6) aus dem Hydrogelmaterial (1), wobei das Hydrogelmaterial (1) ein aus anionisch geladenen Bausteinen (3) und ungeladenen Bausteinen ausgebildetes Polymernetzwerk (2) ist, dessen Affinität für bioaktive Moleküle (6) anhand von die anionisch geladenen Bausteine (3) definierenden Parametern konfigurierbar ist und eine elektrisch leitfähige Komponente (5) aufweist, deren elektrischer Widerstand und elektrische Ladungsspeicherkapazität von einer Interaktion mit den Hydogel-Bausteinen (3) und einer Bindung von bioaktiven Molekülen (6) an das Hydrogelmaterial (1) abhängt, wobei die elektrisch leitfähige Komponente (5) geeignet ist, die anionische Ladung des Hydrogelmaterials (1) und dessen Affinität für bioaktive Moleküle (6) durch den Einfluss eines elektrischen Potentials zu verändern, bei dem Verfahren das Hydrogelmaterial (1) mit einem Biofluid in Kontakt gebracht wird, wobei eine Änderung des elektrischen Widerstandes und/oder eine Änderung der Ladungsspeicherkapazität des Hydrogelmaterials (1) erfasst und anhand der erfassten Änderung des elektrischen Widerstandes und/oder der erfassten Ladungsspeicherkapazitätsänderung eine Aufnahme von bioaktiven Molekülen (6) in das Hydrogelmaterial (1) oder eine Freisetzung von bioaktiven Molekülen (6) aus dem Hydrogelmaterial (1) in das Biofluid ermittelt wird, und/oder wobei eine Konzentration von bioaktiven Molekülen (6) in dem Biofluid und/oder eine Konzentration von bioaktiven Molekülen (6) in dem Hydrogelmaterial (1) durch ein auf das Hydrogelmaterial (1) wirkendes elektrisches Potential beeinflusst wird.

2. Verfahren nach Anspruch 1, **dadurch gekennzeichnet, dass** zur Freisetzung von bioaktiven Molekülen (6) das Hydrogelmaterial (1) vor der Kontaktierung mit dem Biofluid mit einer vorgegebenen Konzentration eines vorgegebenen bioaktiven Moleküls (6) beladen wird.

3. Verfahren nach Anspruch 1 oder 2, **dadurch gekennzeichnet, dass** das Polymernetzwerk (2) in seiner Zusammensetzung anhand von mindestens drei die anionisch geladenen Bausteine (3) definierenden Parametern, ausgewählt aus einer Gruppe von Parametern P0, P1, P2, P3, konfigurierbar ist, wobei der Parameter P0 einem Wert aus der Anzahl der ionisierten, anionischen Gruppen, unter Annahme einer 30%igen Ionisierung aller anionischen Gruppen, je Volumeneinheit des unter physiologischen Bedingungen gequollenen Hydrogelmaterials (1) entspricht, der Parameter P1 einem Wert aus der Anzahl der stark anionischen Gruppen, mit einem intrinsischen pKs-Wert kleiner als 2,5, je Volumeneinheit des unter physiologischen Bedingungen gequollenen Hydrogelmaterials (1) entspricht, der Parameter P2 einem Wert aus der Anzahl der stark anionischen Gruppen, mit einem intrinsischen pKs-Wert kleiner als 2,5, je Wiederholeinheit geteilt durch die Molmasse der Wiederholeinheit entspricht und der Parameter P3 einem Wert zur Beschreibung der Amphiphilie der anionischen Bausteine (3) entspricht, wobei ein elektrischer Widerstand und/oder eine elektrische Ladungsspeicherkapazität des Hydrogelmaterials (1) durch Parameterwerte einer Parameterkonfiguration des Hydrogelmaterials (1) vorgegeben wird.

**4.** Verfahren nach Anspruch 1 bis 3, **dadurch gekennzeichnet, dass** zur Erfassung einer Bindung von bioaktiven Molekülen (6) eine Veränderung der Impedanz des Hydrogelmaterials (1) bei zumindest einer Frequenz im Bereich von 0,1 Hz bis 1 MHz gemessen wird.

**5.** Verfahren nach einem der Ansprüche 1 bis 4, **dadurch gekennzeichnet, dass** das Hydrogelmaterial (1) zur Aufnahme von bioaktiven Molekülen (6) mit einem elektrischen Potential im Bereich von 1 mV bis 1000 mV, vorzugsweise im Bereich von 400 mV bis 600 mV beaufschlagt wird, und dass das Hydrogelmaterial (1) zur Freigabe von bioaktiven Molekülen mit einem elektrischen Potential im Bereich von -1 mV bis -1000 mV, vorzugsweise in einem Bereich von -400 mV bis -600 mV beaufschlagt wird.

**6.** Verfahren nach einem der Ansprüche 1 bis 5, **dadurch gekennzeichnet, dass** das Hydrogelmaterial (1) zur Aufnahme von bioaktiven Molekülen (6) mit einem konstanten elektrischen Strom größer als 0 mA beaufschlagt wird, wobei die Richtung des elektrischen Stromflusses zur Freigabe von bioaktiven Molekülen (6) geändert wird.

**Claims**

**1.** A method for detecting and influencing an absorption of bioactive molecules (6) in a hydrogel material (1) and/or a release of bioactive molecules (6) from the hydrogel material (1), wherein the hydrogel material (1) is a polymer network (2) that is formed of anionically charged building blocks (3) and uncharged building blocks, has an affinity for bioactive molecules (6) that is configurable by means of parameters that define the anionically charged building blocks (3), and has an electrically conductive component (5), the electrical resistance and electrical charge storage capacity of which depend on an interaction with the hydrogel building blocks (3) and on bonding of bioactive molecules (6) to the hydrogel material (1), wherein the electrically conductive component (5) is suitable for changing the anionic charge of the hydrogel material (1) and its affinity for bioactive molecules (6) by the influence of an electrical potential, in which method the hydrogel material (1) is brought into contact with a biofluid, wherein a change in the electrical resistance and/or a change in the charge storage capacity of the hydrogel material (1) is detected, and an absorption of bioactive molecules (6) into the hydrogel material (1) or a release of bioactive molecules (6) from the hydrogel material (1) into the biofluid is determined on the basis of the detected change in the electrical resistance and/or on the basis of the detected change in the charge storage capacity, and/or wherein a concentration of bioactive molecules (6) in the biofluid and/or a concentration of bioactive molecules (6) in the hydrogel material (1) is influenced by an electrical potential acting on the hydrogel material (1).

**2.** The method according to Claim 1, **characterised in that,** for the release of bioactive molecules (6), the hydrogel material (1) is loaded with a predefined concentration of a predefined bioactive molecule (6) before contact with the biofluid.

**3.** The method according to Claim 1 or 2, **characterised in that** the polymer network (2) is configurable in composition by means of at least three parameters that define the anionically charged building blocks (3), selected from a group of parameters P0, P1, P2, P3, wherein parameter P0 corresponds to a value of the number of the ionised, anionic groups, assuming 30% ionisation of all anionic groups, per unit volume of the hydrogel material (1) swollen under physiological conditions, parameter P1 corresponds to a value of the number of the highly anionic groups, with an intrinsic pKs value of less than 2.5, per unit volume of the hydrogel material (1) swollen under physiological conditions, parameter P2 corresponds to a value of the number of the highly anionic groups, with an intrinsic pKs value of less than 2.5, per repeating unit divided by the molar mass of the repeating unit, and parameter P3 corresponds to a value for describing the amphiphilia of the anionic building blocks (3), wherein an electrical resistance and/or an electrical charge storage capacity of the hydrogel material (1) is predefined by parameter values of a parameter configuration of the hydrogel material (1).

**4.** The method according to Claim 1 to 3, **characterised in that,** for the detection of bonding of bioactive molecules (6), a change in the impedance of the hydrogel material (1) is measured at at least one frequency in the range of 0.1 Hz to 1 MHz.

**5.** The method according to one of Claims 1 to 4, **characterised in that,** for the absorption of bioactive molecules (6), an electrical potential in the range of 1 mV to 1000 mV, preferably in the range of 400 mV to 600 mV, is applied to the hydrogel material (1), and that, for the release of bioactive molecules, an electrical potential in the range of -1 mV to -1000 mV, preferably in a range of -400 mV to -600 mV, is applied to the hydrogel material (1).

6. The method according to one of Claims 1 to 5, **characterised in that,** for the absorption of bioactive molecules (6), a constant electrical current of greater than 0 mA is applied to the hydrogel material (1), wherein the direction of the flow of electrical current is changed to release bioactive molecules (6).

**Revendications**

1. Procédé destiné à détecter et à influencer l'absorption de molécules bioactives (6) par un matériau hydrogel (1) et/ou à libérer des molécules bioactives (6) contenues dans le matériau hydrogel (1), dans lequel le matériau hydrogel (1) est un réseau polymère (2) qui est formé de composants chargés anioniquement (3) ainsi que de composants non chargés, dont l'affinité pour les molécules bioactives (6) peut être configurée à l'aide de paramètres définissant les composants chargés anioniquement (3) et qui présente un composant électriquement conducteur (5) dont la résistance électrique et la capacité de stockage de charge électrique dépendent d'une interaction avec les composants de l'hydrogel (3) ainsi que d'une liaison de molécules bioactives (6) audit matériau hydrogel (1), dans lequel le composant électriquement conducteur (5) est capable de modifier la charge anionique du matériau hydrogel (1) ainsi que son affinité pour les molécules bioactives (6) sous l'influence d'un potentiel électrique, procédé dans lequel le matériau hydrogel (1) est mis en contact avec un biofluide, dans lequel une modification de la résistance électrique et/ou une modification de la capacité de stockage de charge du matériau hydrogel (1) est détectée et, sur la base de la modification de la résistance électrique détectée et/ou de la modification de la capacité de stockage de charge détectée, une absorption de molécules bioactives (6) par le matériau hydrogel (1) ou une libération de molécules bioactives (6) contenues dans le matériau hydrogel (1) vers le biofluide est alors déterminée, et/ou dans lequel une concentration de molécules bioactives (6) dans le biofluide et/ou une concentration de molécules bioactives (6) dans le matériau hydrogel (1) est influencée par un potentiel électrique agissant sur le matériau hydrogel (1).

2. Procédé selon la revendication 1, **caractérisé en ce que** pour permettre la libération de molécules bioactives (6), le matériau hydrogel (1) doit être tout d'abord chargé de molécules bioactives (/d'une molécule bioactive) (6) à une concentration prédéfinie avant d'entrer en contact avec le biofluide.

3. Procédé selon la revendication 1 ou 2, **caractérisé en ce que** la composition du réseau polymère (2) peut être configurée à l'aide d'au moins trois paramètres définissant les composants (3) chargés anioniquement et choisis parmi un groupe de paramètres PO, P1, P2, P3, le paramètre PO correspondant à une valeur représentant le nombre des groupes anioniques ionisés, en supposant une ionisation de 30 % de tous les groupes anioniques, par unité de volume du matériau hydrogel (1) gonflé dans des conditions physiologiques, le paramètre P1 correspondant à une valeur représentant le nombre des groupes fortement anioniques, avec une valeur pKs intrinsèque inférieure à 2,5, par unité de volume du matériau hydrogel (1) gonflé dans des conditions physiologiques, le paramètre P2 correspondant à une valeur représentant le nombre des groupes fortement anioniques, avec une valeur pKs intrinsèque inférieure à 2,5, par unité répétitive divisée par la masse molaire de l'unité répétitive, et le paramètre P3 correspondant à une valeur décrivant l'amphiphilie des composants anioniques (3), une résistance électrique et/ou une capacité de stockage de charge électrique du matériau hydrogel (1) étant prédéfinie(s) par des valeurs paramétriques d'une configuration paramétrique du matériau hydrogel (1).

4. Procédé selon l'une quelconque des revendications 1 à 3, **caractérisé en ce qu'**une modification de l'impédance du matériau hydrogel (1) est mesurée à au moins une fréquence comprise entre 0,1 Hz et 1 MHz en vue de détecter une liaison de molécules bioactives (6).

5. Procédé selon l'une quelconque des revendications 1 à 4, **caractérisé en ce que** pour permettre l'absorption de molécules bioactives (6), le matériau hydrogel (1) est soumis à un potentiel électrique compris entre 1 mV et 1000 mV, de préférence entre 400 mV et 600 mV, et **en ce que** pour permettre la libération de molécules bioactives, le matériau hydrogel (1) est soumis à un potentiel électrique compris entre -1 mV et -1000 mV, de préférence entre -400 mV et -600 mV.

6. Procédé selon l'une quelconque des revendications 1 à 5, **caractérisé en ce que** pour permettre l'absorption de molécules bioactives (6), le matériau hydrogel (1) est soumis à un courant électrique constant supérieur à 0 mA, la direction du flux de courant électrique étant changée pour permettre la libération de molécules bioactives (6).

Fig. 1

A: Bildung des Netzwerk 1

anionische Polymere + Vernetzer-molekül = Netzwerk 1

P1,P2,P3

B: Bildung des Netzwerk 2 im Netzwerk 1

Netzwerk 1 + Leitfähige monomere/Polymere = leitfähiges Hydrogel

C: Dotierung des leitfähigen Polymer im Netzwerk 2 durch die anionischen Gruppen im Netzwerk 1

P1/P2 } Bestimmt durch anionische Polymere
P3 }

C: Elektrische Ladungsmodulation im leitfähigen Polymer

- 1V ← 0 V → + 1V

0    +1    +3

EP 4 251 221 B1

Fig. 2

biomolekular

Stimulation

Detektion

elektrostatisch

Signalprotein

elektrodynamisch

1

5

6

5

4

7

elektrisch

Poly(ethylenglykol) (PEG)-Peptid-Konjugat

4

Poly(ethylendioxythiophen) (PEDOT)

5

sulfatierte und sulfonierte (Bio-)Polymere

3

## Fig. 3

Nicht-poröses
Hydrogelmaterial

Poröses
Hydrogelmaterial

100 µm

100 µm

EP 4 251 221 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018162009 A2
- US 20190390068 A1
- US 9299476 B2
- US 20200191757 A